# EUROPÄISCHE PATENTSCHRIFT

(11) **EP 1 313 432 B1**
(45) Veröffentlichungstag und Bekanntmachung des Hinweises auf die Patenterteilung: **09.03.2005**
(21) Anmeldenummer: 01965197.5
(22) Anmeldetag: 17.08.2001
(51) Int. Cl.: A61K 7/06, A61K 7/48

(54) **VERWENDUNG HYDROPHILER PFROPFCOPOLYMERE MIT N-VINYLAMIN- UND/ODER OFFENKETTIGEN N-VINYLAMIDEINHEITEN IN KOSMETISCHEN FORMULIERUNGEN**
USE OF HYDROPHILIC GRAFT COPOLYMERS WITH N-VINYLAMINE AND/OR OPEN-CHAINED N-VINYLAMIDE UNITS IN COSMETIC FORMULATIONS
UTILISATION DE COPOLYMERES GREFFES A MOTIFS N-VINYLAMINE ET/OU N-VINYLAMIDE A CHAINE OUVERTE DANS DES FORMULATIONS COSMETIQUES

(30) Priorität: 22.08.2000 DE 10041211
(43) Veröffentlichungstag der Anmeldung: 28.05.2003
(73) Patentinhaber: BASF AKTIENGESELLSCHAFT, 67056 Ludwigshafen (DE)
(72) Erfinder: SCHNEIDER, Tanja, 64625 Bensheim (DE); GOTSCHE, Michael, 68167 Mannheim (DE); NEGELE, Anton, 67146 Deidesheim (DE); NGUYEN KIM, Son, 69502 Hemsbach (DE); FRENZEL, Stefan, 68161 Mannheim (DE); WOOD, Claudia, 69469 Weinheim (DE)
(86) Internationale Anmeldenummer: PCT/EP2001/009491
(87) Internationale Veröffentlichungsnummer: WO 2002/015854

(56) Entgegenhaltungen:
- EP-A- 0 629 649
- EP-A- 0 931 537
- WO-A-98/25981
- DE-A- 19 640 363
- DE-A- 19 907 587

## Beschreibung

Die Erfindung betrifft die Verwendung von Pfropfcopolymerisaten als Bestandteil in kosmetischen Mitteln. Die Pfropfcopolymerisate entstehen dabei durch Pfropfung von monoethylenisch ungesättigten, offenkettigen N-Vinylamideinheiten enthaltenden Monomeren auf eine polymere Pfropfgrundlage.

Polymere finden in Kosmetik und Medizin vielfache Anwendung. In Seifen, Cremes und Lotionen beispielsweise dienen sie in der Regel als Formulierungsmittel, z.B. als Verdicker, Schaumstabilisator oder Wasserabsorbens oder auch dazu, die reizende Wirkung anderer Inhaltsstoffe abzumildern oder die dermale Applikation von Wirkstoffen zu verbessern. Ihre Aufgabe in der Haarkosmetik dagegen bestehen darin, die Eigenschaften des Haares zu beeinflussen.

So werden Conditioner dazu eingesetzt, um die Trocken- und Nasskämmbarkeit, Gefühl, Glanz und Erscheinungsform zu verbessern sowie dem Haar antistatische Eigenschaften zu verleihen. Bevorzugt werden wasserlösliche Polymere mit polaren, häufig kationischen Funktionalitäten eingesetzt, die eine größere Affinität zur strukturell bedingten negativen Oberfläche des Haares aufweisen. Struktur und Wirkungsweise verschiedener Haarbehandlungspolymere sind in Cosmetic & Toiletries 103 (1988) 23 beschrieben. Handelsübliche Conditionerpolymere sind z.B. kationische Hydroxyethylcellulose, kationische Polymere auf der Basis von N-Vinylpyrrolidon, z.B. Copolymere aus N-Vinylpyrrolidon und quarterniertem N-Vinylimidazol, Acrylamid und Diallyldimethylammoniumchlorid oder Silikone.

Zur Festigung von Haarfrisuren werden Vinyllactam-Homo- und Copolymere und Carboxylatgruppenhaltige Polymere eingesetzt. Anforderungen an Haarfestigerharze sind zum Beispiel eine starke Festigung bei hoher Luftfeuchtigkeit, Elastizität, Auswaschbarkeit vom Haar, Verträglichkeit in der Formulierung und ein angenehmer Griff des Haares.

Schwierigkeiten bereitet oft die Kombination verschiedener Eigenschaften wie zum Beispiel starke Festigung und angenehmer Griff des Haares.

Die WO-A-96/03969 beschreibt Haarpflegemittel, enthaltend ein N-Vinylformamid-Homopolymer oder ein Copolymer aus N-Vinylformamideinheiten und einem weiteren Vinylmonomer, ausgewählt unter Styrolen, Alkylestern von Acryl- und Methacrylsäure; Vinylestern der Formel CH₂=CH-OCO-Alkyl, N-Alkyl-substituierten Acryl- und Methacrylamiden, Estern von Fumar-, Ithacon- und Maleinsäure, Vinylethern, Hydroxy-funktionalisierten Acrylaten und Methacrylaten, Acrylamid, nicht-Alkyl-substituierten Acrylamiden und cyclischen Amiden. Als konkretes Beispiel für ein cyclisches Amid wird N-Vinylpyrrolidon genannt. Weitere Beispiele für Vinylmonomere sind sekundäre, tertiäre und quarternäre Amine, wie Dimethyl-diallylammoniumchlorid, Dimethylaminoethylmethacrlyat oder Dimethylaminopropyl-methacrylat.

DE 19640363 beschreibt Copolymere aus N-Vinylformamid und quarterniertem N-Vinylimidazol sowie deren Anwendungen in der Kosmetik.

DE 19907587.5 beschreibt die Verwendung von Polymerisaten, die erhältlich sind durch radikalische Polymerisation von mindestens einem Vinylester in Gegenwart von polyetherhaltigen Verbindungen und gegebenenfalls eines oder mehreren copolymerisierbaren Monomeren und anschließender zumindest teilweiser Verseifung der Esterfunktion in haarkosmetischen Formulierungen. Als copolymerisierbare Monomere ist u.a. Vinylformamid genannt.

Die DE-A1-44 09 903 beschreibt N-Vinyleinheiten enthaltende Pfropfpolymerisate, Verfahren zu ihrer Herstellung und ihre Verwendung. Dabei werden auf einer Pfropfgrundlage, welches ein Polymerisat ist, das jeweils mindestens 5 Gew.-% Einheiten der Formeln 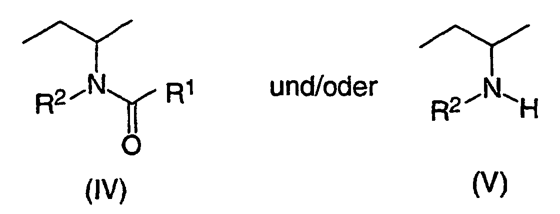 enthält, wobei R¹, R² = H oder C₁- bis C₆-Alkyl bedeuten, monoethylenisch ungesättigte Monomere aufgepfropft. Als monoethylenisch ungesättigte Monomere kommen alle ethylenisch ungesättigten Monomere in Frage, deren Polymerisation durch die Amingruppen in freier oder in Salzform nicht inhibiert wird, wie z.B. monoethylenisch ungesättigte Mono- und Dicarbonsäuren, deren Salze und Ester mit C₁- bis C₃₀-Alkoholen. Eine Eignung dieser Pfropfcopolymere als Wirkstoff in kosmetischen Formulierungen wird nicht genannt.

Die WO 96/34903 beschreibt N-Vinyleinheiten enthaltende Pfropfpolymerisate, Verfahren zu ihrer Herstellung und ihre Verwendung. Dabei werden auf einer Pfropfgrundlage, welches ein Polymerisat ist, das mindestens 3 Einheiten eines C₂- bis C₄-Alkylenoxids enthalten, und/oder Polytetrahydrofuran enthalten, monoethylenisch ungesättigte Monomere aufgepfropft und anschließend zumindest teilweise verseift. Eine Eignung dieser Pfropfcopolymere als Wirkstoff in kosmetischen Formulierungen wird nicht genannt.

Aus der US-A-5 334 287 sind Pfropfpolymerisate bekannt, die durch radikalisch initiierte Polymerisation von N-Vinylcarbonsäureamiden, vorzugsweise N-Vinylformamid, und gegebenenfalls anderen Monomeren in Gegenwart von Monosacchariden, Oligosacchariden, Polysacchariden oder jeweils deren Derivaten und gegebenenfalls Hydrolyse der einpolymerisierten Gruppe N-Vinylcarbonsäureamide unter Bildung von Vinylamineinheiten erhältlich sind. Eine Eignung dieser Pfropfcopolymere als Wirkstoff in kosmetischen Formulierungen wird nicht genannt.

In der WO 9825981 werden amphiphile Pfropfpolymere durch Pfropfen hydrophober Monomerer, wie z.B. Styrol, auf Polymere die Strukturelemente der Formel (IV) und/oder (V) enthalten synthetisiert. Die dabei erhaltenen Pfropfpolymeren werden u.a. als Zusatzstoffe zu kosmetischen Formulierungen verwendet.

In der DE-A1-196 40 363 wird die Verwendung wasserlöslicher Copolymere als Wirkstoff in kosmetischen Formulierungen beansprucht. Als charakteristisches Strukturelement enthält das Copolymer Einheiten der allgemeinen Formel (VI) und 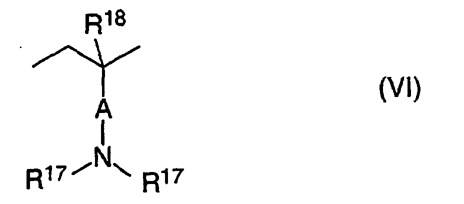 worin A für eine chemische Bindung oder eine Alkylengruppe steht, die Reste R¹⁷ unabhängig voneinander für H, Alkyl, Cycloalkyl, Aryl oder Aralkyl und R¹⁸ für H, Alkyl oder Aralkyl stehen.

Körperpflegecremes, die ein Monoaldehyd-modifiziertes Vinylaminpolymer enthalten, sind aus der US 5 270 379 bekannt.

Copolymere, die beispielsweise als Haarfestiger Verwendung finden und aufgebaut sind aus N-Vinvlamidmonomeren der Formel 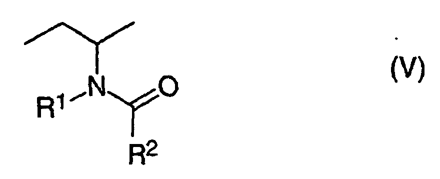 worin R1 und R2 für H oder C₁-C₅-Alkyl stehen und das Comonomer ausgewählt ist unter Vinylethern, Vinyllactamen, Vinylhalogeniden, Vinylestern einbasiger gesättigter Carbonsäuren, (Meth)acrylsäureestern, -amiden und -nitrilen und estern, Anhydriden und Imiden der Maleinsäure sind aus der DE 14 95 692 bekannt.

In der US 4 713 236 sind Haarconditioner auf Basis von Vinylamineinheiten enthaltenden Polymeren beschrieben. Genannt sind dabei insbesondere Polyvinylamin sowie dessen Salze, α-substituierte Polyvinylamine wie z.B. Poly-(α-aminoacrylsäure) oder auch Copolymere, die neben Vinylamin Comonomere wie Vinylalkohol, Acrylsäure, Acrylamid, Maleinsäureanhydrid, Vinylsulfonat und 2-Acrylamido-2-methylpropan-sulfonsäure einpolymerisiert enthalten.

Aufgabe der vorliegenden Erfindung war es Polymere zu finden, die für kosmetische Anwendungen gut geeignet sind und beispielsweise im Gebiet Haarkosmetik gute anwendungstechnische Eigenschaften wie angenehmer Griff und gleichzeitig gute Konditionierwirkung bzw. eine gute Festigungswirkung aufweisen.

Die Aufgabe wurde erfindungsgemäß gelöst durch Verwendung von hydrophilen Pfropfcopolymerisaten, die erhältlich sind durch radikalische Pfropfcopolymerisation von
a) mindestens einer offenkettigen N-Vinylamidverbindung der allgemeinen Formel (I) 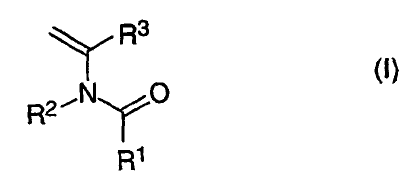 wobei R¹, R², R³ = H oder C₁- bis C₆-Alkyl bedeuten, und
b) gegebenenfalls eines oder mehreren weiteren copolymerisierbaren Monomeren
auf eine polymere Pfropfgrundlage c)
für kosmetische Anwendungen.

Bei der Herstellung der erfindungsgemäß verwendeten Polymerisate kann es während der Polymerisation zu einer Pfropfung auf die polymere Pfropfgrundlage c) kommen, was zu den vorteilhaften Eigenschaften der Polymerisate führen kann. Es sind jedoch auch andere Mechanismen als Pfropfung vorstellbar.

Je nach Pfropfungsgrad sind unter den erfindungsgemäß verwendeten Polymerisaten sowohl reine Pfropfpolymerisate als auch Mischungen der o.g. Pfropfpolymerisate mit ungepfropften Verbindungen c) und Homo- oder Copolymerisaten der Monomeren a) und b) zu verstehen.

Unter wasserlöslichen Polymeren sollen hier Polymere verstanden werden, die sich zu mindestens 1 g/l bei 20°C in Wasser lösen. Unter wasserdispergierbaren Polymeren sollen hier Polymere verstanden werden, die unter Rühren in dispergierbare Partikel zerfallen.

Zur Herstellung der erfindungsgemäß verwendeten Polymerisate werden als offenkettige N-Vinylamidverbindung a) der allgemeinen Formel (I) beispielsweise folgende Monomere eingesetzt: N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinyl-butyramid. Aus dieser Gruppe von Monomeren verwendet man vorzugsweise N-Vinylformamid.

Selbstverständlich können auch Mischungen der jeweiligen Monomeren aus der Gruppe a) wie z.B. Mischungen aus N-Vinylformamid und N-Vinylacetamid, copolymerisiert werden.

Die polymere Pfropfgrundlage c) wird bevorzugt ausgewählt aus
c1) polyetherhaltigen Verbindungen
c2) Polymerisaten, die mindestens 5 Gew.-% an Vinylpyrrolidoneinheiten einpolymerisiert enthalten
c3) Polymerisaten, die mindestens 50 Gew.-% an Vinylalkohol-Einheiten enthalten
c4) natürliche Substanzen c4), die Saccharid-Strukturen enthalten

Als polyetherhaltige Verbindung c1) können sowohl Polyalkylenoxide auf Basis von Ethylenoxid, Propylenoxid, Butylenoxid und weiteren Alkylenoxiden als auch Polyglycerin verwendet werden. Je nach Art der Monomerbausteine enthalten die Polymere folgende Struktureinheiten.
-(CH₂)₂-O-, -(CH₂)₃-O-, -(CH₂)₄-O-, -CH₂-CH(R⁹)-O-, -CH₂-CHOR¹⁰-CH₂-O-
mit
- R⁹: C₁-C₂₄-Alkyl;
- R¹⁰: Wasserstoff, C₁-C₂₄-Alkyl, R⁹-C(=O)-, R⁹-NH-C(=O)-.

Dabei kann es sich bei den Struktureinheiten sowohl um Homopolymere als auch um statistische Copolymere und Blockcopolymere handeln.

Bevorzugt werden als Polyether (b) Polymerisate der allgemeinen Formel II verwendet, mit einem Molekulargewicht >300 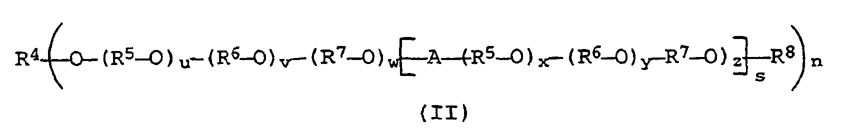 in der die variablen unabhängig voneinander folgende Bedeutung haben:
- R⁴: Wasserstoff, C₁-C₂₄-Alkyl, R⁹-C(=O)-, R⁹-NH-C(=O)-, Polyalkoholrest;
- R⁸: Wasserstoff, C₁-C₂₄-Alkyl, R⁹-C(=O)-, R⁹-NH-C (=O) -;
- R⁵ bis R⁷: -(CH₂)₂-, -(CH₂)₃-, -(CH₂)₄-, -CH₂-CH(R⁹)-, -CH₂-CHOR¹⁰-CH₂-;
- R⁹: C₁-C₂₄-Alkyl;
- R¹⁰: Wasserstoff, C₁-C₂₄-Alkyl, R⁹-C(=O)-, R⁹-NH-C(=O)-;
- A: -C(=O)-O, -C(=O)-B-C(=O)-O, -C(=O)-NH-B-NH-C(=O)-O;
- B: -(CH₂)ₜ-, Arylen, ggf . substituiert;
- n: 1 bis 1000;
- s: 0 bis 1000;
- t: 1 bis 12;
- u: 1 bis 5000;
- v: 0 bis 5000;
- w: 0 bis 5000:
- x: 0 bis 5000;
- y: 0 bis 5000;
- z: 0 bis 5000.

Die endständigen primären Hydroxylgruppen der auf Basis von Polyalkylenoxiden hergestellten Polyether sowie die sekundären OH-Gruppen von Polyglycerin können dabei sowohl in ungeschützter Form frei vorliegen als auch mit Alkoholen einer Kettenlänge C₁-C₂₄ bzw. mit Carbonsäuren einer Kettenlänge C₁-C₂₄ verethert bzw. verestert werden oder mit Isocyanaten zu Urethanen umgesetzt werden.

Als Alkylreste für R⁴ und R⁸ bis R¹⁰ seien verzweigte oder unverzweigte C₁-C₂₄-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als bevorzugte Vertreter der oben genannten Alkylreste seien verzweigte oder unverzweigte C₁-C₁₂-, besonders bevorzugt C₁-C₆-Alkylketten genannt.

Das Molekulargewicht der Polyether liegt im Bereich größer 300 (nach Zahlenmittel), bevorzugt im Bereich von 300 bis 100000, besonders bevorzugt im Bereich von 500 bis 50000, ganz besonders bevorzugt im Bereich von 800 bis 40000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 mol-%, der Propylenoxidanteil 1 bis 60 mol-% und der Anteil an Butylenoxid in den Copolymerisaten 1 bis 30 mol-%. Neben geradkettigen können auch verzweigte Homo- oder Copolymerisate als polyetherhaltige Verbindungen b) verwendet werden.

Verzweigte Polymerisate können hergestellt werden, indem man beispielsweise an Polyalkoholresten, z.B. an Pentaerythrit, Glycerin oder an Zuckeralkoholen wie D-Sorbit und D-Mannit aber auch an Polysaccharide wie Cellulose und Stärke, Ethylenoxid und gegebenenfalls noch Propylenoxid und/oder Butylenoxide anlagert. Die Alkylenoxid-Einheiten können im Polymerisat statistisch verteilt sein oder in Form von Blöcken vorliegen.

Es ist aber auch möglich, Polyester von Polyalkylenoxiden und aliphatischen oder aromatischen Dicarbonsäuren, z.B. Oxalsäure, Bernsteinsäure, Adipinsäure und Terephthalsäure mit Molmassen von 1500 bis 25000, wie z.B. beschrieben in EP-A-0 743 962, als polyetherhaltige Verbindung zu verwenden. Des weiteren können auch Polycarbonate durch Umsetzung von Polyalkylenoxiden mit Phosgen oder Carbonaten wie z.B. Diphenylcarbonat, sowie Polyurethane durch Umsetzung von Polyalkylenoxiden mit aliphatischen und aromatischen Diisocyanaten verwendet werden.

Besonders bevorzugt werden als Polyether c1) Polymerisate der allgemeinen Formel II mit einem mittleren Molekulargewicht von 300 bis 100.000 (nach dem Zahlenmittel), in der die Variablen unabhängig voneinander folgende Bedeutung haben:
- R⁴: Wasserstoff, C₁-C₁₂-Alkyl, R⁹-C(=O)-, R⁹-NH-C(=O)-, Polyalkoholrest;
- R⁸: Wasserstoff, C₁-C₁₂-Alkyl, R⁹-C(=O)-, R⁹-NH-C(=O)-;
- R⁵ bis R⁷: -(CH₂)₂-, -(CH₂)₃-, -(CH₂)₄-, -CH₂-CH(R⁹)-, -CH₂-CHOR¹⁰-CH₂-;
- R⁹: C₁-C₁₂-Alkyl;
- R¹⁰: Wasserstoff, C₁-C₁₂-Alkyl, R⁹-C(=O)-, R⁹-NH-C(=O)-;
- n: 1 bis 8;
- s: 0;
- u: 2 bis 2000;
- v: 0 bis 2000;
- w: 0 bis 2000.

Ganz besonders bevorzugt werden als Polyether b) Polymerisate der allgemeinen Formel II mit einem mittleren Molekulargewicht von 500 bis 50000 (nach dem Zahlenmittel), in der die Variablen unabhängig voneinander folgende Bedeutung haben:
- R⁴: Wasserstoff, C₁-C₆-Alkyl, R⁹-C(=O)-, R⁹-NH-C(=O)-;
- R⁸: Wasserstoff, C₁-C₆-Alkyl, R⁹-C(=O)-, R⁹-NH-C(=O)-;
- R⁵ bis R⁷: -(CH₂)₂-, -(CH₂)₃-, -(CH₂)₄-, -CH₂-CH(R⁹)-, -CH₂-CHOR¹⁰-CH₂-;
- R⁹: C₁-C₆-Alkyl;
- R¹⁰: Wasserstoff, C₁-C₆-Alkyl, R⁹-C(=O)-. R⁹-NH-C(=O)-;
- n: 1;
- s: 0;
- u: 5 bis 500;
- v: 0 bis 500;
- w: 0 bis 500.

Als Polyether können jedoch auch Silikonderivate eingesetzt werden. Geeignete Silikonderivate sind die unter dem INCI Namen Dimethicone Copolyole oder Silikontenside bekannten Verbindungen wie zum Beispiel die unter den Markennamen Abil® (der Fa. T. Goldschmidt), Alkasil® (der Fa. Rhöne-Poulenc), Silicone Polyol Copolymer® (der Fa Genesee), Belsil® (der Fa. Wacker), Silwet® (der Fa. Witco, Greenwich, CT, USA) oder Dow Corning (der Fa. Dow Corning) erhältlich. Diese beinhalten Verbindungen mit den CAS-Nummern 64365-23-7; 68937-54-2; 68938-54-5; 68937-55-3.

Silikone werden in der Haarkosmetik im allgemeinen zur Verbesserung des Griffs eingesetzt. Die Verwendung von polyetherhaltigen Silikonderivaten als Polyether (b) in den erfindungsgemäßen Polymeren kann deshalb zusätzlich zu einer Verbesserung des Griffs der Haare führen.

Bevorzugte Vertreter solcher polyetherhaltigen Silikonderivaten sind solche, die die folgenden Strukturelemente enthalten: 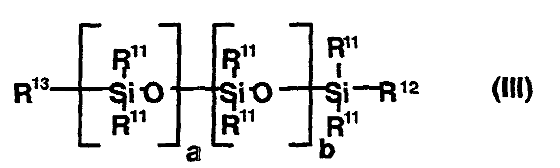 wobei :
- R¹² **=**: CH₃ oder 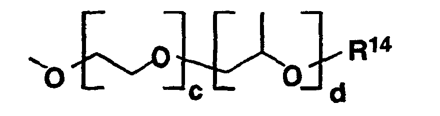
- R¹³ =: CH₃ oder R¹²
- R¹⁴ =: H, CH₃, 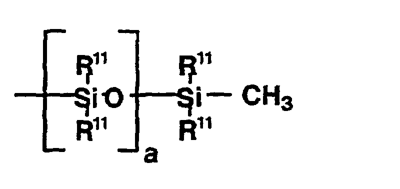 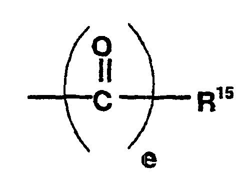
- R¹⁵: ein organischer Rest aus 1 bis 40 Kohlenstoffatomen, der Amino-, Carbonsäure- oder Sulfonatgruppen enthalten kann oder für den Fall e=0, auch das Anion einer anorganischen Säure bedeutet,
und wobei die Reste R¹¹ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich R¹² sind, wobei: 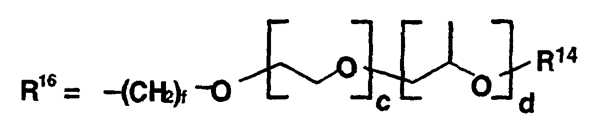 mit der Maßgabe, daß mindestens einer der Reste R¹¹, R¹² oder R¹³ ein polyalkylenoxidhaltiger Rest nach obengenannter Definition ist,
und f eine ganze Zahl von 1 bis 6 ist,
a und b ganze Zahlen derart sind, daß das Molekulargewicht des Polysiloxan-Blocks zwischen 300 und 30000 liegt,
c und d ganze Zahlen zwischen 0 und 50 sein können mit der Maßgabe, daß die Summe aus c und d größer als 0 ist, und e 0 oder 1 ist.

Bevorzugte Reste R¹² und R¹⁶ sind solche, bei denen die Summe aus c+d zwischen 5 und 30 beträgt.

Bevorzugt werden die Gruppen R¹¹ aus der folgenden Gruppe ausgewählt: Methyl, Ethyl, Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen, speziell Phenyl oder Naphthyl, gemischt aromatisch-aliphatische Reste wie Benzyl oder Phenylethyl sowie Tolyl und Xylyl und R¹⁶.

Besonders geeignete Reste R¹⁴ sind solche, bei denen im Falle von R¹⁴ = -(CO)ₑ-R¹⁵ R¹⁵ ein beliebiger Alkyl-, Cycloalkyl oder Arylrest bedeutet, der zwischen 1 und 40 C-Atomen besitzt und der weitere ionogene Gruppen wie NH₂, COOH, SO₃H tragen kann.

Bevorzugte anorganische Reste R¹⁵ sind, für den Fall e=0, Phosphat und Sulfat.

Besonders bevorzugte polyetherhaltige Silikonderivate sind solche der allgemeinen Struktur:

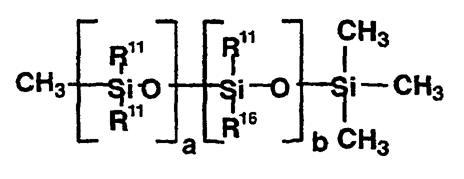

Des weiteren können als Polyether (c1) auch Homo- und Copolymerisate aus polyalkylenoxidhaltigen ethylenisch ungesättigten Monomeren wie beispielsweise Polyalkylenoxid(meth)acrylate, Polyalkylenoxidvinylether, Polyalkylenoxid(meth)acrylamide, Polyalkylenoxidallyamide oder Polyalkylenoxidvinylamide verwendet werden. Selbstverständlich können auch Copolymerisate solcher Monomere mit anderen ethylenisch ungesättigten Monomeren eingesetzt werden.

Als polyetherhaltige Verbindungen c1) können aber auch Umsetzungsprodukte von Polyethyleniminen mit Akylenoxiden eingesetzt werden. Als Alkylenoxide werden in diesem Fall bevorzugt Ethylenoxid, Propylenoxid, Butylenoxid und Mischungen aus diesen, besonders bevorzugt Ethylenoxid verwendet. Als Polyethylenimine können Polymere mit zahlenmittleren Molekulargewichten von 300 bis 20000, bevorzugt 500 bis 10000, ganz besonders bevorzugt 500 bis 5000, eingesetzt werden. Das Gewichtsverhältnis zwischen eingesetztem Alkylenoxid und Polyethylenimin liegt im Bereich von 100 : 1 bis 0,1 : 1, bevorzugt im Bereich 50 : 1 bis 0,5 : 1, ganz besonders bevorzugt im Bereich 20 : 1 bis 0,5 : 1.

Als Pfropfgrundlage können jedoch auch Polymerisate c2), die mindestens 5 Gew.-% an Vinylpyrrolidon-Einheiten enthalten, eingesetzt werden. Bevorzugt enthalten diese als Pfropfgrundlage eingesetzten Polymerisate einen Vinylpyrrolidon-Anteil von mindestens 10 Gew.-%, ganz besonders bevorzugt von mindestens 30 Gew.-%.

Als Comonomere des Vinylpyrrolidons zur Synthese der Pfropfgrundlage (c2) kommen beispielsweise N-Vinylcaprolactam, N-Vinylimidazol, N-Vinyl-2-methylimidazol, N-Vinyl-4-methylimidazol, 3-Methyl-1-vinylimidazoliumchlorid, 3-Methyl-1-vinylimidazoliummethylsulfat, Diallylammoniumchlorid, Styrol, Alkylstyrole in Frage.

Weitere geeignete Comonomere zur Herstellung der Pfropfgrundlage c3) sind beispielsweise sind monoethylenisch ungesättigten c₃-C₆-Carbonsäuren wie z.B. Acrylsäure, Methacrylsäure, Crotonsäure, Fumarsäure, sowie deren Ester, Amide und Nitrile wie z.B. Acrylsäuremethylester, Acrylsäureethylester, Methacrylsäuremethylester, Methacrylsäureethylester, Methacrylsäurestearylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäureiethylester, 2-Ethylhexylacrylat, 2-Ethylhexylmethacrylat, Maleinsäureanhydrid sowie dessen Halbester, Alkylenglykol(meth)acrylate, Acrylamid, Methacrylamid, N-Dimethylacrylamid, N-tert.-butylacrylamid, Acrylnitril, Methacrylnitril, Vinylether wie z.B. Methyl-, Ethyl-, Butyl oder Dodecylvinylether, kationische Monomere wie Dialkylaminoalkyl(meth)acrylate und Dialkylaminoalkyl(meth)acrylamide wie Dimethylaminothylacrylat, Diethylaminoethylacrylat, Diethylaminoethylmethacrylat, sowie die Salze der zuletzt genannten Monomeren mit Carbonsäuren oder Mineralsäuren sowie die quarternierten Produkte.

Die Herstellung der Pfropfgrundlage erfolgt nach bekannten Verfahren, zum Beispiel der Lösungs-, Fällungs-, Suspensions- oder Emulsionspolymerisation unter Verwendung von Verbindungen, die unter den Polymerisationsbedingungen Radikale bilden. Die Polymerisationstemperaturen liegen üblicherweise in dem Bereich von 30 bis 200, vorzugsweise 40 bis 110°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, zum Beispiel Natriumsulfit, Natriumbisulfit, Natriumformaldehydsulfoxilat und Hydrazin. Diese Systeme können gegebenenfalls zusätzlich noch geringe Mengen eines Schwermetallsalzes enthalten.

Die Homo- und Copolymeren (Pfropfgrundlage C2) besitzen K-Werte von mindestens 7, vorzugsweise 10 bis 250. Die Polymeren können jedoch K-Werte bis zu 300 haben. Die K-Werte werden bestimmt nach H. Fikentscher, Cellulose-Chemie, Band 13, 58 bis 64 und 71 bis 74 (1932) in wässriger Lösung bei 25°C, bei Konzentrationen, die je nach K-Wert-Bereich zwischen 0,1 % und 5 % liegen.

Als Propfgrundlage können jedoch auch Polymerisate c3), die mindestens 50 Gew.-% an Vinylalkoholeinheiten besitzen. Bevorzugt enthalten diese Polymerisate mindestens 70 Gew.-%, ganz besonders bevorzugt 80 Gew.-% Polyvinylalkoholeinheiten. Solche Polymerisate werden überlicherweise durch Polymerisation eines Vinylesters und anschließender zumindest teilweiser Alkoholyse, Aminolyse oder Hydrolyse hergestellt. Bevorzugt sind Vinylester linearer und verzweigter C₁-C₁₂-Carbonsäuren, ganz besonders bevorzugt ist Vinylacetat. Die Vinylester können selbstverständlich auch im Gemisch eingesetzt werden.

Als Comonomere des Vinylesters zur Synthese der Pfropfgrundlage (c3) kommen beispielsweise N-Vinylcaprolactam, N-Vinylpyrrolidon, N-Vinylimidazol, N-Vinyl-2-methylimidazol, N-Vinyl-4-methylimidazol, 3-Methyl-1-vinylimidazoliumchlorid, 3-Methyl-1-vinylimidazoliummethylsulfat, Diallylammoniumchlorid, Styrol, Alkylstyrole in Frage.

Weitere geeignete Comonomere zur Herstellung der Pfropfgrundlage c3) sind beispielsweise sind monoethylenisch ungesättigten c₃-C₆-Carbonsäuren wie z.B. Acrylsäure, Methacrylsäure, Crotonsäure, Fumarsäure, sowie deren Ester, Amide und Nitrile wie z.B. Acrylsäuremethylester, Acrylsäureethylester, Methacrylsäuremethylester, Methacrylsäureethylester, Methacrylsäurestearylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäureiethylester, 2-Ethylhexylacrylat, 2-Ethylhexylmethacrylat, Maleinsäureanhydrid sowie dessen Halbester, Alkylenglykol(meth)acrylate, Acrylamid, Methacrylamid, N-Dimethylacrylamid, N-tert.-butylacrylamid, Acrylnitril, Methacrylnitril, Vinylether wie z.B. Methyl-, Ethyl-, Butyl oder Dodecylvinylether, kationische Monomere wie Dialkylaminoalkyl(meth)acrylate und Dialkylaminoalkyl(meth)acrylamide wie Dimethylaminothylacrylat, Diethylaminoethylacrylat, Diethylaminoethylmethacrylat, sowie die Salze der zuletzt genannten Monomeren mit Carbonsäuren oder Mineralsäuren sowie die quarternierten Produkte.

Bevorzugt Propfgrundlagen c3) sind Polymerisate, die durch Homopolymerisation von Vinylacetat und anschließender zumindest teilweiser Hydrolyse, Alkoholyse oder Aminolyse hergestellt werden.

Die Herstellung der Pfropfgrundlage c3) erfolgt nach bekannten Verfahren, zum Beispiel der Lösungs-, Fällungs-, Suspensions- oder Emulsionspolymerisation unter Verwendung von Verbindungen, die unter den Polymerisationsbedingungen Radikale bilden. Die Polymerisationstemperaturen liegen üblicherweise in dem Bereich von 30 bis 200, vorzugsweise 40 bis 110°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, zum Beispiel Natriumsulfit, Natriumbisulfit, Natriumformaldehydsulfoxilat und Hydrazin. Diese Systeme können gegebenenfalls zusätzlich noch geringe Mengen eines Schwermetallsalzes enthalten.

Zur Herstellung der Pfropfgrundlage c3) werden die Estergruppen der ursprünglichen Monomere und gegebenenfalls weiterer Monomere nach der Polymerisation durch Hydrolyse, Alkoholyse oder Aminolyse zumindest teilweise gespalten. Im nachfolgenden wird dieser Verfahrensschritt allgemein als Verseifung bezeichnet. Die Verseifung erfolgt in an sich bekannter Weise durch Zugabe einer Base oder Säure, bevorzugt durch Zugabe einer Natrium- oder Kaliumhydroxidlösung in Wasser und/oder Alkohol. Besonders bevorzugt werden methanolische Natrium- oder Kaliumhydroxidlösungen eingesetzt, Die Verseifung wird bei Temperaturen im Bereich von 10 bis 80°C, bevorzugt im Bereich von 20 bis 60°C, durchgeführt. Der Verseifungsgrad hängt ab von der Menge der eingesetzten Base bzw. Säure, von der Verseifungstemperatur, der Verseifungszeit und dem Wassergehalt der Lösung.

Besonders bevorzugte Propfgrundlagen c3) sind Polymerisate, die durch Homopolymerisation von Vinylacetat und anschließender zumindest teilweiser Verseifung hergestellt werden. Solche Polyvinylalkoholeinheiten enthaltenden Polymere sind unter dem Namen Mowiol® erhältlich. Als Pfopfgrundlage können aber auch natürliche Substanzen c4), die Saccharid-Strukturen enthalten, eingesetzt werden. Solche natürlichen Substanzen sind beispielsweise Saccharide pflanzlicher oder tierischer Herkunft oder Produkte, die durch Metabolisierung durch Mikroorganismen entstanden sind, sowie deren Abbauprodukte. Geeignete Pfropfgrundlagen c4) sind beispielsweise Oligosaccharide, Polysaccharide, oxidativ, enzymatisch oder hydrolytisch abgebaute Polysaccharide, oxidativ hydrolytisch abgebaute oder oxidativ enzymatisch abgebaute Polysaccharide, chemisch modifizierte Oligo- oder Polysaccharide und Mischungen davon.

Bevorzugte Produkte sind die in US 5,334,287 auf Spalte 4 Zeile. 20 bis Spalte 5 Zeile 45 genannten Verbindungen.

Die bevorzugten zusätzlich eingesetzten ethylenisch ungesättigten Comonomere (b) können durch die folgende allgemeine Formel beschrieben werden:

X-C (O) CR²⁰=CHR¹⁹

wobei
X ausgewählt ist aus der Gruppe der Reste -OH, -OM, -OR²¹, NH₂, -NHR²¹, N(R²¹)₂;
M ist ein Kation ausgewählt aus der Gruppe bestehend aus: Na⁺, K⁺, Mg⁺⁺, Ca⁺⁺, Zn⁺⁺, NH₄⁺, Alkylammonium, Dialkylammonium, Trialkylammonium und Tetraalkylammonium;
die Reste R²¹ können identisch oder verschieden ausgewählt werden aus der Gruppe bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.

R²⁰ und R¹⁹ sind unabhängig voneinander ausgewählt aus der Gruppe bestehend aus: -H, C₁-C₈ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren (b) sind zum Beispiel Acrylsäure oder Methacrylsäure und deren Salze, Ester und Amide. Die Salze können von jedem beliebigen nicht toxischen Metall, Ammonium oder substituierten Ammonium-Gegenionen abgeleitet sein.

Die Ester können abgeleitet sein von C₁-C₄₀ linearen, C₃-C₄₀ verzweigtkettigen oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin und 1,2,6-Hexantriol, von Aminoalkoholen oder von Alkoholethern wie Methoxyethanol und Ethoxyethanol, (Alkyl)Polyethylenglykolen, (Alkly)Polypropylenglykolen oder ethoxylierten Fettalkoholen, beispielsweise C₁₂-C₂₄-Fettalkoholen umgesetzt mit 1 bis 200 Ethylenoxid-Einheiten.

Ferner eignen sich N,N-Dialkylaminoalkylacrylate- und -methacrylate und N-Dialkylaminoalkylacryl- und -methacrylamide der allgemeinen Formel (VII) 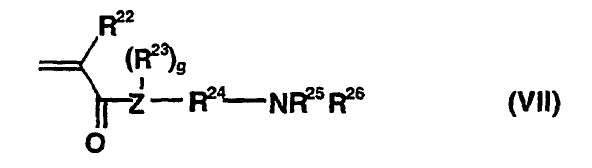 mit
- R²² =: H, Alkyl mit 1 bis 8 C-Atomen,
- R²³ =: H, Methyl,
- R²⁴ =: Alkylen mit 1 bis 24 C-Atomen, optional substituiert durch Alkyl,
- R²⁵, R²⁶ =: C₁-C₄₀ Alkylrest,
- Z =: Stickstoff für g = 1 oder Sauerstoff für g = 0

Die Amide können unsubstituiert, N-Alkyl oder N-Alkylamino monosubstituiert oder N,N-dialkylsubstituiert oder N,N-dialkylaminodisubstituiert vorliegen, worin die Alkyl- oder Alkylaminogruppen von C₁-C₄₀ linearen, C₃-C₄₀ verzweigtkettigen, oder C₃-C₄₀ carbocyclischen Einheiten abgeleitet sind. Zusätzlich können die Alkylaminogruppen quaternisiert werden.

Bevorzugte Comonomere der Formel VII sind N,N-Dimethylaminomethyl(meth)acrylat, N,N-Diethylaminomethyl(meth)acrylat, N,N-Dimethylaminoethyl(meth)acrylat, N,N-Diethylaminoethyl(meth)acrylat. N-[3-(dimethylamino)propyl]methacrylämid und N-[3-(dimethylamino)propyl]acrylamid.

Ebenfalls verwendbare Comonomere (b) sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus C₁-C₄ Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Comonomere (b) sind Allylester von C₁-C₄₀ linearen, C₃-C₄₀ verzweigtkettigen oder C₃-C₄₀ carbocyclische Carbonsäuren, Vinyl- oder Allylhalogenide, bevorzugt Vinylchlorid und Allylchlorid, Vinylether, bevorzugt Methyl-, Ethyl-, Butyl- oder Dodecylvinylether, Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel VIII geeignet, worin R²⁷ bis R²⁹ unabhängig voneinander für Wasserstoff, C₁-C₄-Alkyl oder Phenyl steht:

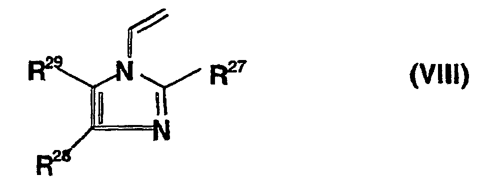

Weitere geeignete Comonomere (b) sind Diallylamine der allgemeinen Formel (IX) 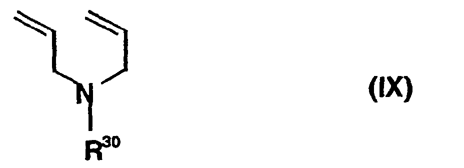 mit R³⁰ = C₁- bis C₂₄-Alkyl

Weitere geeignete Comonomere (b) sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.
Besonders geeignete Comonomere (b) sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, Stearyl(meth)acrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren wie zum Beispiel Acrylamidopropansulfonsäure;
Acrylamid, Methacrylamid, Ethacrylamid, N-Methylacrylamid, N,N-Dimethylacrylamid, N-Ethylacrylamid, N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylvinylimidazol, N,N-Dimethylaminomethyl(meth)acrylat, N,N-Diethylaminomethyl(meth)acrylat, N,N-Dimethylaminoethyl(meth)acrylat, N,N-Diethylaminoethyl(meth)acrylat, N,N-Dimethylaminobutyl(meth)acrylat, N,N-Diethylaminobutyl (meth) acrylat, N,N-Dimethylaminohexyl(meth)acrylat, N,N-Dimethylaminooctyl(meth)acrylat, N,N-Dimethylaminododecyl(meth)acrylat, N-[3-(dimethylamino)propyl]methacrylamid, N-[3-(dimethylamino)propyl]acrylamid, N-[3-(dimethylamino)butyl]methacrylamid. N-[8-(dimethylamino)octyl]methacrylamid, N-[12-(dimethylamino)dodecyl]methacrylamid, N-[3-(diethylamino)propyl)methacrylamid, N-[3-(diethylamino)propyl]acrylamid;
Maleinsäure, Fumarsäure, Maleinsäureanhydrid und seine Halbester, Crotonsäure, Itaconsäure, Diallyldimethylammoniumchlorid, Vinylether (zum Beispiel: Methyl-, Ethyl-, Butyl- oder Dodecylvinylether), Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylate, Styrol, ungesättigte Sulfonsäuren wie zum Beispiel Acrylamidopropansulfonsäure, Vinylpyrrolidon, Vinylcaprolactam, Vinylether (z.B.: Methyl-, Ethyl-, Butyl- oder Dodecylvinylether), 1-Vinylimidazol, 1-Vinyl-2-methylimidazol, N,N-Dimethylaminomethylmethacrylat und N-[3-(dimethylamino)propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)propyl]methacrylamid quaternisiert mit Methylchlorid, Methylsulfat oder Diethylsulfat.

Monomere, mit einem basischen Stickstoffatom, können dabei auf folgende Weise quarternisiert werden:

Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Amine kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.

Die Quaternisierung kann vor der Polymerisation oder nach der Polymerisation durchgeführt werden.

Außerdem können die Umsetzungsprodukte von ungesättigten Säuren, wie z.B. Acrylsäure oder Methacrylsäure, mit einem quaternisierten Epichlorhydrin der allgemeinen Formel (X) eingesetzt werden (R³¹ = C₁- bis C₄₀-Alkyl).

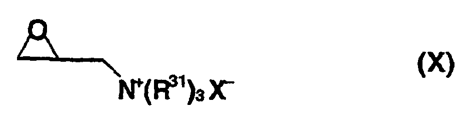

Beispiele hierfür sind zum Beispiel:
(Meth)acryloyloxyhydroxypropyltrimethylammoniumchlorid und
(Meth)acryloyloxyhydroxypropyltriethylammoniumchlorid.

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Zusätzlich zu den oben genannten Comonomeren können als Comonomere (b) sogenannte Makromonomere wie zum Beispiel silikonhaltige Makromonomere mit ein oder mehreren radikalisch polymerisierbaren Gruppen oder Alkyloxazolinmakromonomere eingesetzt werden, wie sie zum Beispiel in der EP 408 311 beschrieben sind.

Des weiteren können fluorhaltige Monomere, wie sie beispielsweise in der EP 558423 beschrieben sind, vernetzend wirkende oder das Molekulargewicht regelnde Verbindungen in Kombination oder alleine eingesetzt werden.

Als Regler können die üblichen dem Fachmann bekannten Verbindungen, wie zum Beispiel Schwefelverbindungen (z.B.: Mercaptoethanol, 2-Ethylhexylthioglykolat, Thioglykolsäure oder Dodecylmercaptan), sowie Tribromchlormethan oder andere Verbindungen, die regelnd auf das Molekulargewicht der erhaltenen Polymerisate wirken, verwendet werden.

Es können gegebenenfalls auch thiolgruppenhaltige Silikonverbindungen eingesetzt werden.
Bevorzugt werden silikonfreie Regler eingesetzt.

Als vernetzende Monomere können Verbindungen mit mindestens zwei ethylenisch ungesättigten Doppelbindungen eingesetzt werden, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 2,4-Bis(hydroxymethyl)cyclohexan, Hydroxypivalinsäure-neopentylglycolmonoester, 2,2-Bis(4-hydroxyphenyl)propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Ferner geeignet sind Amide von ungesättigten Carbonsäuren, wie z.B., Acryl- und Methacrylsäure, Itaconsäure, Maleinsäure, und N-Allylaminen von mindestens zweiwertigen Aminen, wie zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin oder entsprechende Ammoniumsalze, z.B. Triallylmethylammoniumchlorid oder -methylsulfat, als Vernetzer geeignet.

Weiterhin können N-Vinylverbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff eingesetzt werden.

Weitere geeignete Vernetzer sind Divinyldioxan, Tetraallylsilan oder Tetravinylsilan.

Besonders bevorzugte Vernetzer sind beispielsweise Methylenbisacrylamid, Divinylbenzol, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind, sowie Allyl- oder Vinylether von mehrwertigen Alkoholen, beispielsweise 1,2-Ethandiol, 1,4-Butandiol, Diethylenglykol, Trimethylolpropan, Glycerin, Pentaerythrit, Sorbitan und Zucker wie Saccharose, Glucose, Mannose.

Ganz besonders bevorzugt als Vernetzer sind Pentaerythrittriallylether, Allylether von Zuckern wie Saccharose, Glucose, Mannose, Divinylbenzol, Methylenbisacrylamid, N,N'-Divinylethylenharnstoff, und (Meth-)Acrylsäureester von Glykol, Butandiol, Trimethylolpropan oder Glycerin oder (Meth)Acrylsäureester von mit Ethylenoxid und/oder Epichlorhydrin umgesetzten Glykol, Butandiol, Trimethylolpropan oder Glycerin.

Der Anteil der vernetzend wirkenden Monomeren beträgt 0 bis 10 Gew.-%, bevorzugt 0 bis 5 Gew.-%, ganz besonders bevorzugt 0 bis 2 Gew.-%.

Bei der Polymerisation zur Herstellung der erfindungsgemäßen Polymerisate können gegebenenfalls auch andere Polymere, wie zum Beispiel Polyamide, Polyurethane, Polyester, Homo- und Copolymere von ethylenisch ungesättigten Monomeren, zugegen sein. Beispiele für solche zum Teil auch in der Kosmetik eingesetzten Polymeren sind die unter den Handelsnamen bekannten Polymere Amerhold™, Ultrahold™, Ultrahold Strong™, Luviflex™ VBM, Luvimer™, Acronal™, Acudyne™, Stepanhold™, Lovocryl™, Versatyl™, Amphomer™ oder Eastma AQ™.

Die erfindungsgemäßen Comonomere (b) können, sofern sie ionisierbare Gruppen enthalten, vor oder nach der Polymerisation, zum Teil oder vollständig mit Säuren oder Basen neutralisiert werden, um so zum Beispiel die Wasserlöslichkeit oder -dispergierbarkeit auf ein gewünschtes Maß einzustellen.

Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-Propyl]amin, 2-Amino-2-Methyl-1,3-Propandiol, 2-Amino-2-hydroxymethyl-1,3-Propandiol sowie Diamine, wie zum Beispiel Lysin, verwendet werden.

Zur Herstellung der Polymerisate können die Monomeren der Komponente a) und gegebenenfalls der Komponente B) in Gegenwart der Pfopfgrundlage c) sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidonopropan)dihydrochlorid oder 2-2'-Azo-bis-(2-methylbutyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat.

Bevorzugt werden organische Peroxide eingesetzt.

Die Polymerisation kann auch durch Einwirkung von ultravioletter Strahlung, gegebenenfalls in Gegenwart von UV-Initiatoren, durchgeführt werden. Für das Polymerisieren unter Einwirkung von UV-Strahlen setzt man die dafür üblicherweise in Betracht kommenden Photoinitiatoren bzw. Sensibilisatoren ein. Hierbei handelt es sich beispielsweise um Verbindungen wie Benzoin und Benzoinether, α-Methylbenzoin oder α-Phenylbenzoin. Auch sogenannte Triplett-Sensibilisatoren, wie Benzyldiketale, können verwendet werden. Als UV-Strahlungsquellen dienen beispielsweise neben energiereichen UV-Lampen, wie Kohlenbogenlampen, Quecksilberdampflampen oder Xenonlampen auch UV-arme Lichtquellen, wie Leuchtstoffröhren mit hohem Blauanteil.

Die verwendeten Mengen an Initiator bzw. Initiatorgemischen bezogen auf eingesetztes Monomer liegen zwischen 0,01 und 10 Gew.-%, vorzugsweise zwischen 0,1 und 5 Gew.-%.

Die Polymerisation erfolgt im Temperaturbereich von 40 bis 200°C, bevorzugt im Bereich von 50 bis 140°C, besonders bevorzugt im Bereich von 60 bis 110°C. Sie wird üblicherweise unter atmosphärischem Druck durchgeführt, kann jedoch auch unter vermindertem oder erhöhtem Druck, vorzugsweise zwischen 1 und 5 bar, ablaufen.

Die Polymerisation kann beispielsweise als Lösungspolymerisation, Polymerisation in Substanz, Emulsionspolymerisation, umgekehrte Emulsionspolymerisation, Suspensionspolymerisation, umgekehrte Suspensionspolymerisation oder Fällungspolymerisation durchgeführt werden, ohne daß die verwendbaren Methoden darauf beschränkt sind.

Bei der Polymerisation in Substanz kann man so vorgehen, daß man die Pfopfgrundlage c) in mindestens einem Monomer der Gruppe a) und eventuell weiteren Comonomeren der Gruppe b) löst und nach Zugabe eines Polymerisationsinitiators die Mischung auspolymerisiert. Die Polymerisation kann auch halbkontinuierlich durchgeführt werden, indem man zunächst einen Teil, z.B. 10 % des zu polymerisierenden Gemisches aus der Pfropfgrundlage c), mindestens einem Monomeren der Gruppe a), eventuell weiteren Comonomeren der Gruppe b) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Polymerisate können auch dadurch erhalten werden, daß man die Pfopfgrundlag c) in einem Reaktor vorlegt, auf die Polymerisationstemperatur erwärmt und mindestens ein Monomer der Gruppe a), eventuell weiteren Comonomeren der Gruppe b) und Polymerisationsinitiator entweder auf einmal, absatzweise oder vorzugsweise kontinuierlich zufügt und polymerisiert.

Falls gewünscht, kann die oben beschriebene Polymerisation auch in einem Lösemittel durchgeführt werden. Geeignete Lösemittel sind beispielsweise Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Die Polymerisation kann auch in Wasser als Lösemittel durchgeführt werden. In diesem Fall liegt zunächst eine Lösung vor, die in Abhängigkeit von der Menge der zugegebenen Monomeren der Komponente a) in Wasser mehr oder weniger gut löslich ist. Um wasserunlösliche Produkte, die während der Polymerisation entstehen können, in Lösung zu überführen, kann man beispielsweise organische Lösemittel zusetzen, wie einwertige Alkohole mit 1 bis 3 Kohlenstoffatomen, Aceton oder Dimethylformamid. Man kann jedoch auch bei der Polymerisation in Wasser so verfahren, daß man die wasserunlöslichen Polymerisate durch Zugabe üblicher Emulgatoren oder Schutzkolloide, z.B. Polyvinylalkohol, in eine feinteilige Dispersion überführt.

Als Emulgatoren verwendet man beispielsweise ionische oder nichtionische Tenside, deren HLB-Wert im Bereich von 3 bis 13 liegt. Zur Definition des HLB-Werts wird auf die Veröffentlichung von W.C. Griffin, J. Soc. Cosmetic Chem., Band 5, 249 (1954), hingewiesen.

Die Menge an Tensiden, bezogen auf das Polymerisat, beträgt 0,1 bis 10 Gew.-%. Bei Verwendung von Wasser als Lösemittel erhält man Lösungen bzw. Dispersionen der Polymerisate. Sofern man Lösungen des Polymerisates in einem organischen Lösemittel herstellt bzw. in Mischungen aus einem organischen Lösemittel und Wasser, so verwendet man pro 100 Gew.-Teile des Polymerisates 5 bis 2000, vorzugsweise 10 bis 500 Gew.-Teile des organischen Lösemittels oder des Lösemittelgemisches.

Bevorzugt sind Polymere, die erhältlich sind durch radikalische Pfropfcopolymerisation von

| | | |
|---|---|---|
| a) | 10 - 90 Gew.-% | mindestens einer offenkettigen N-Vinylamidverbindung der allgemeinen Formel I und |
| b) | 0 - 60 Gew.-% | eines oder mehreren weiteren copolymerisierbaren Monomeren auf |
| c) | 10 - 90 Gew.-% | einer wasserlöslichen oder wasserdispergierbaren polymeren Pfropfgrundlage |

Bevorzugt sind Polymere, die erhältlich sind durch radikalische Pfropfcopolymerisation von

| | | |
|---|---|---|
| a) | 20 - 80 Gew.-% | mindestens einer offenkettigen N-Vinylamidverbindung der allgemeinen Formel I und |
| b) | 0 - 60 Gew.-% | eines oder mehreren weiteren copolymerisierbaren Monomeren auf |
| c) | 20 - 80 Gew.-% | einer oder mehrerer wasserlöslicher oder wasserdispergierbarer polymeren Pfropfgrundlage |

Ganz besonders bevorzugt sind Polymere, die erhältlich sind durch radikalische Pfropfcopolymerisation von

| | | |
|---|---|---|
| a) | 40 - 80 Gew.-% | mindestens einer offenkettigen N-Vinylamidverbindung der allgemeinen Formel I und |
| b) | 0 - 40 Gew.-% | eines oder mehreren weiteren copolymerisierbaren Monomeren auf |
| c) | 20 - 80 Gew.-% | einer oder mehrerer wasserlöslicher oder wasserdispergierbarer polymeren Pfropfgrundlage |

Die erfindungsgemäßen Pfropfcopolymerisate können im Anschluss an die Polymerisation verseift werden. Durch die Verseifung wird eine kationische Gruppe im Polymer erzeugt. Dies kann zu einer erhöhten Wasserlöslichkeit und verbesserten konditionierenden Eigenschaften in kosmetischen Anwendungen führen.

Aus den oben beschriebenen Pfropfcopolymerisaten erhält man durch teilweise oder vollständige Abspaltung der Formylgruppen oder der C₁- bis C₆-Alkyl-C=O-Gruppen, aus denen in das Polymerisat eingebauten offenkettigen N-Vinylamiden (IV), unter Bildung von Amin- bzw. Ammoniumgruppen Einheiten der Formel (V)

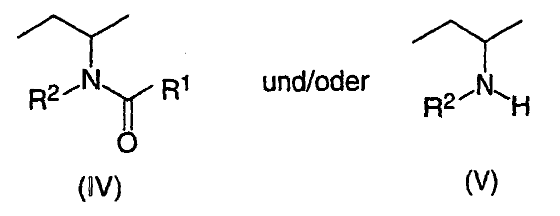

In den Formeln (IV) und (V) haben die Substituenten R¹ und R² jeweils die oben angegebene Bedeutung. In Abhängigkeit von den bei der Hydrolyse gewählten Reaktionsbedingungen enthält man entweder eine partielle oder vollständige Hydrolyse der Einheiten (IV).

Enthält die Pfropfgrundlage neben den hydrolyseunempfindlichen Vinylpyrrolidoneinheiten außerdem Comonomere, die hydrolyseempfindlich sind, wie z.B. Vinylacetat oder Acrylamid, so findet auch in der Pfropfgrundlage eine Hydrolyse statt. So reagiert Vinylacetat zu Vinylalkoholgruppen und Acrylamid zu Acrylsäuregruppen.

Als Hydrolysemittel eignen sich Mineralsäuren, wie Halogenwasserstoffe, die gasförmig oder in wässriger Lösung eingesetzt werden können. Vorzugsweise verwendet man Salzsäure, Schwefelsäure, Salpetersäure und Phosphorsäure sowie organische Säuren, wie C₁- bis C₅-Carbonsäüren und aliphatische oder aromatische Sulfonsäuren. Pro Formylgruppenäquivalent, das aus den einpolymerisierten Einheiten (IV) abgespalten werden soll, benötigt man 0,05 bis 2, vorzugsweise 1 bis 1,5 Moläquivalente einer Säure.
Die Hydrolyse der einpolymerisierten Einheiten der Struktur (IV) kann auch mit Hilfe von Basen vorgenommen werden, z.B. von Metallhydroxiden, insbesondere von Alkalimetall- und Erdalkalimetallhydroxiden. Vorzugsweise verwendet man Natriumhydroxid oder Kaliumhydroxid. Die Hydrolyse kann gegebenenfalls auch in Gegenwart von Ammoniak oder Aminen durchgeführt werden.
Die Hydrolyse im sauren oder im alkalischen pH-Bereich erfolgt z.B. bei Temperaturen von 30 bis 170, vorzugsweise 50 bis 120°C. Sie ist nach etwa 2 bis 8, vorzugsweise 3 bis 5 Stunden beendet. Nach diesen Reaktionszeiten erreicht man Hydrolysegrade der Einheiten der einpolymerisierten Monomeren der Formel (I) von 1 bis 100 %. Besonders bewährt hat sich eine Verfahrensweise, bei der zur Hydrolyse die Basen oder Säuren in wässriger Lösung zugesetzt werden. Nach der Hydrolyse führt man im allgemeinen eine Neutralisation durch, so dass der pH-Wert der hydrolysierten Polymerlösung 2 bis 8, vorzugsweise 3 bis 7 beträgt. Die Neutralisation ist dann erforderlich, wenn ein Fortschreiten der Hydrolyse von teilweise hydrolysierten Polymerisaten vermieden oder verzögert werden soll. Die Hydrolyse kann auch mit Hilfe von Enzymen vorgenommen werden.

Die so hergestellten Polymerisate können durch Umsetzung von im Polymer vorhandenen Hydroxyl- und/oder Aminofunktionen mit Epoxiden der Formel X nachträglich kationisiert werden (R³¹ = C₁ bis C₄₀ Alkyl) .

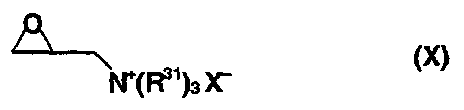

Dabei können bevorzugt die Hydroxylgruppen der Polyvinylalkohol-Einheiten und Vinylamin-Einheiten, entstanden durch Hydrolyse von Vinylformamid, mit den Epoxiden umgesetzt werden.
Die Epoxide der Formel X können auch in situ durch Umsetzung der entsprechenden Chlorhydrine mit Basen, beispielsweise Natriumhydroxid, erzeugt werden.
Bevorzugt wird 2,3-Epoxypropyl-trimethylammoniumchlorid bzw. 3-Chlor-2-hydroxypropyl-trimethylammoniumchlorid eingesetzt.
Die K-Werte der Polymerisate sollen im Bereich von 10 bis 300, bevorzugt 25 bis 250, besonders bevorzugt 25 bis 200, ganz besonders bevorzugt im Bereich von 30 und 150, liegen. Der jeweils gewünschte K-Wert läßt sich in an sich bekannter Weise durch die Zusammensetzung der Einsatzstoffe einstellen. Die K-Werte werden bestimmt nach Fikentscher, Cellulosechemie, Bd. 13, S. 58 bis 64, und 71 bis 74 (1932) in N-Methylpyrrolidon bei 25°C und Polymerkonzentrationen, die je nach K-Wert-Bereich zwischen 0,1 Gew.-% und 5 Gew.-% liegen.

Zur Entfernung von Lösungsmitteln können die Polymerlösungen wasserdampfdestilliert werden. Nach der Wasserdampfdestillation erhält man je nach Wahl der Komponenten a-c wässrige Lösungen oder Dispersionen.

Die erhaltenen Polymerisate können auch nachträglich vernetzt werden, indem man die Hydroxylgruppen bzw. Aminogruppen im Polymer mit mindestens bifunktionellen Reagentien umsetzt. Bei niedrigen Vernetzungsgraden erhält man wasserlösliche Produkte, bei hohen Vernetzungsgrade wasserquellbare bzw. unlösliche Produkte.

Beispielsweise können die erfindungsgemäßen Polymerisate mit Dialdehyden und Diketonen, z.B. Glyoxal, Glutaraldehyd, Succindialdehyd oder Terephthalaldehyd, umgesetzt werden. Desweiteren eignen sich aliphatische oder aromatische Carbonsäuren, beispielsweise Maleinsäure, Oxalsäure, Malonsäure, Succinsäure oder Citronensäure, bzw. Carbonsäurederivaten wie Carbonsäureester, -anhydride oder -halogenide. Ferner sind mehrfunktionelle Epoxide geeignet, z.B. Epichlorhydrin, Glycidylmethacrylat, Ethylenglykoldiglycidylether, 1,4-Butandioldiglycidylether oder 1,4-Bis(glycidyloxy)benzol. Ferner eigenen sich Diisocyanate, beispielsweise Hexamethylendiisocyanat, Isophorondiisocyanat, Methylendiphenyldiisocyanat, Toluylendiisocyanat oder Divinylsulfon.

Weiterhin eignen sich anorganische Verbindungen wie Borsäure oder Borsäuresalze, beispielsweise Natriummetaborat, Borax (Dinatriumtetraborat), sowie Salze mehrwertiger Kationen, z.B. Kupfer(II)salze wie Kupfer(II)acetat oder Zink-, Aluminium-, Titansalze.

Borsäure bzw. Borsäuresalze wie Natriummetaborat oder Dinatriumtetraborat eignen sich bevorzugt zur nachträglichen Vernetzung. Dabei können die Borsäure bzw. Borsäuresalze, bevorzugt als Salzlösungen, den Lösungen der erfindungsgemäßen Polymerisate zugegeben werden. Bevorzugt werden die Borsäure bzw. Borsäuresalze den wässerigen Polymerisatlösungen hinzugefügt.
Die Borsäure bzw. Borsäuresalze können den Polymerlösungen direkt nach der Herstellung zugefügt werden. Es ist aber auch möglich, die Borsäure bzw. Borsäuresalze nachträglich den kosmetischen Formulierungen mit den erfindungsgemäßen Polymerisaten zuzusetzen, bzw. während des Herstellungsprozesses der kosmetischen Formulierungen.
Der Anteil Borsäure bzw. Borsäuresalze bezogen auf die erfindungsgemäßen Polymere beträgt 0 bis 15 Gew.-%, bevorzugt 0 bis 10 Gew.-%, besonders bevorzugt 0 bis 5 Gew.-%.

Die Polymerisatlösungen und -dispersionen können durch verschiedene Trocknungsverfahren wie z.B. Sprühtrocknung, Fluidized Spray Drying, Walzentrocknung oder Gefriertrocknung in Pulverform überführt werden. Als Trocknungsverfahren wird bevorzugt die Sprühtrocknung eingesetzt. Aus dem so erhaltenen Polymer-Trockenpulver läßt sich durch Lösen bzw. Redispergieren in Wasser erneut eine wäßrige Lösung bzw. Dispersion herstellen. Die Überführung in Pulverform hat den Vorteil einer besseren Lagerfähigkeit, einer einfacheren Transportmöglichkeit sowie eine geringere Neigung für Keimbefall.

Die erfindungsgemäßen wasserlöslichen oder wasserdispergierbaren Ppfopfcopolymerisate hervorragend zur Verwendung in kosmetischen Formulierungen.
Die erfindungsgemäßen Polymere, eignen sich als Stylingmittel und/oder Konditioniermittel in haarkosmetischen Zubereitungen wie Haarkuren, Haarlotionen, Haarspülungen, Haaremulsionen, Spitzenfluids, Egalisierungsmittel für Dauerwellen, "Hot-Oil-Treatment"-Präparate, Conditioner, Festigerlotionen oder Haarsprays. Je nach Anwendungsbegiet können die haarkosmetischen Zubereitungen als Spray, Schaum, Gel, Gelspray oder Mousse appliziert werden.

Die erfindungsgemäßen haarkosmetischen Formulierungen enthalten in einer bevorzugten Ausführungsform
a) 0,05 - 20 Gew.-% des erfindungsgemäßen Polymeren
b) 20 - 99,95 Gew.-%Wasser und/oder Alkohol
c) 0 - 79,5 Gew.-%weitere Bestandteile

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Viskositätsregulierer, Gelbildner, Farbstoffe, Salze, Feuchthaltemittel, Rückfetter und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane (Luviset® P.U.R.) und Polyharnstoffe. Besonders geeignete Polymere sind Copolymere aus t-Butylacrylat, Ethylacrylat, Methacrylsäure (z.B. Luvimer® 100P), Copolymere aus N-tert.-Butylacrylamid, Ethylacrylat, Acrylsäure (Ultrahold® 8, strong), Copolymere aus Vinylacetat, Crotonsäure und gegebenenfall weiteren Vinylestern (z.B. Luviset® Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol® VBM).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam N-Vinylpyrrolidon/N-Vinylimidazoliumsalzen (Luviquat® Hold); kationische Cellulosederivate (Polyquaternium-4 und -10), Acrylamidcopolymere (Polyquaternium-7).

Als weitere Haarkosmetik-Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymere eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten diese Zubereitungen

| | | |
|---|---|---|
| a) | 0,1 - 10 Gew.-% | des erfindungsgemäßen Polymeren |
| b) | 20 - 99,9 Gew.-% | Wasser und/oder Alkohol |
| c) | 0 - 70 Gew.-% | eines Treibmittel |
| d) | 0 - 20 Gew.-% | weitere Bestandteile |

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält

| | | |
|---|---|---|
| a) | 0,1 - 10 Gew.-% | des erfindungsgemäßen Polymeren |
| b) | 55 - 94,8 Gew.-% | Wasser und/oder Alkohol |
| c) | 5 - 20 Gew.-% | eines Treibmittel |
| d) | 0,1 - 5 Gew.-% | eines Emulgators |
| e) | 0 - 10 Gew.-% | weitere Bestandteile |

Als Emulgatoren können alle in Haarschäumen üblicherweise eingesetzten Emulgatoren verwendet werden. Geeignete Emulgatoren können nichtionisch, kationisch bzw. anionisch sein.
Beispiele für nichtionische Emulgatoren (INCI-Nomenklatur) sind Laurethe, z.B. Laureth-4; Cetethe, z.B. Cetheth-1, Polyethylenglycolcetylether; Cetearethe, z.B. Cetheareth-25, Polyglycolfettsäureglyceride, hydroxyliertes Lecithin, Lactylester von Fettsäuren, Alkylpolyglycoside.
Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quaternium-1 bis x (INCI).
Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:

| | | |
|---|---|---|
| a) | 0,1 - 10 Gew.-% | des erfindungsgemäßen Polymeren |
| b) | 60 - 99,85 Gew.-% | Wasser und/oder Alkohol |
| c) | 0,05 - 10 Gew.-% | eines Gelbildners |
| d) | 0 - 20 Gew.-% | weitere Bestandteile |

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Die erfindungsgemäßen Polymere können auch in Shampooformulierungen als Festigungs- und/oder Konditioniermittel eingesetzt werden. Als Konditioniermittel eignen sich insbesondere Polymere mit kationischer Ladung.
Bevorzugte Shampooformulierungen enthalten

| | | |
|---|---|---|
| a) | 0,05 - 10 Gew.-% | des erfindungsgemäßen Polymeren |
| b) | 25 - 94,95 Gew.-% | Wasser |
| c) | 5 - 50 Gew.-% | Tenside |
| d) | 0 - 5 Gew.-% | eines weiteren Konditioniermittels |
| e) | 0 - 10 Gew.-% | weitere kosmetische Bestandteile |

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.
Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.
Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z.B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den erfindungsgemäßen Polymeren eingesetzt werden. Hierzu zählen beispielsweise kationische Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA).

### Synthesebeispiele

Wenn nicht anders angegeben wurden die K-Werte mit 1%igen wässrigen Lösungen bestimmt.

### Beispiel 1:

In einem gerührten Reaktor mit Stickstoffzuführung, Rückflusskühler und Dosiervorrichtung werden 72,8 g Polyethylenglykol mit einem mittleren Molekulargewicht von 4000 (Pluriol E 4000, BASF Aktiengesellschaft), 180 g destilliertes Wasser, 2,8 g 75%ige Phophorsäure und 2,8 g 50%ige Natronlauge vorgelegt und unter Stickstoff bis zum Rückfluss erhitzt. Unter Rückfluss werden 297,1 g Vinylformamid in 1,5 Stunden und 10 g tert.-Butylperoctoat in 32 g Triethylenglycolmonomethylether in 2 Stunden zudosiert und 1,5 Stunden bei dieser Temperatur weiter auspolymerisiert. Da das Reaktionsgemisch im Laufe der Reaktion hochviskos wird 45 Minuten nach Polymerisationsbeginn innerhalb von 1,5 Stunden 250 g destilliertes Wasser zudosiert. Nach Ende der Reaktion wird mit 500 g destilliertem Wasser verdünnt. Die leicht gelbliche Polymerlösung weist einen Festgehalt von 36,3 % und einen K-Wert von 47,4 auf.

### Beispiel 2: Verseifung von Beispiel 1

500 g der in Beispiel 1 erhaltenen Lösung werden mit 100 g destilliertem Wasser und 1 g Natriumpyrosulfit auf 80°C erhitzt. Nach Zugabe von 33 g 25%ige Natronlauge wird 3 Stunden bei 80°C gerührt. Nach Abkühlen wird mit 15 g 38%iger Salzsäure auf pH 8 eingestellt. Die erhaltenen Lösung ist gelblich und leicht trüb.

### Beispiel 3

In einem gerührten Reaktor mit Stickstoffzuführung, Rückflusskühler und Dosiervorrichtung werden 163,8 g Polyethylenglykol mit einem mittleren Molekulargewicht von 9000 (Pluriol E 9000, BASF Aktiengesellschaft) vorgelegt und unter Stickstoffbegasung aufgeschmolzen. Innerhalb von einer Stunde werden 18,5 g N-Vinylformamid und 1,35 g tert.-Butylperoctoat in 16,1 g Triethylenglycolmonomethylether innerhalb von 1,5 Stunden bei 90°C zudosiert. wird eine Stunde nachpolymerisiert. Während der Nachpolymerisation wird das Reaktionsgemisch mit destilliertem Wasser verdünnt. Das erhaltene Polymer weist einen K-Wert von 33,6 auf.

### Beispiel 4

In einem gerührten Reaktor mit Stickstoffzuführung, Rückflusskühler und Dosiervorrichtung werden 127,4 g Polyethylenglykol mit einem mittleren Molekulargewicht von 9000 (Pluriol E 9000, BASF Aktiengesellschaft) aufgeschmolzen. 54,6 g N-Vinylformamid und 70 mg Butandioldivinylether werden innerhalb von einer Stunde und 1,88 g tert.-Butylperoctoat in 16,1 g Triethylenglycolmonomethylether innerhalb von 1,5 Stunden bei 90°C zudosiert und im Anschluss eine Stunde bei dieser Temperatur nachpolymerisiert. Während der Nachpolymerisation wird mit destilliertem Wasser verdünnt. Der K-Wert der leicht gelblichen, klaren Lösung beträgt 41,2.

### Beispiel 5

In einem gerührten Reaktor mit Stickstoffzuführung, Rückflusskühler und Dosiervorrichtung werden 72 g PEG-PPG-Blockcopolymer mit einem mittleren Molekulargewicht von 8000 (Lutrol F 68, BASF Aktiengesellschaft), 180 g destilliertes Wasser, 2,8 g 75%ige Phophorsäure und 2,8 g 50%ige Natronlauge vorgelegt und unter Stickstoff bis zum Rückfluss erhitzt. Unter Rückfluss werden 410 g Vinylformamid in 1,5 Stunden und 10 g tert.-Butylperoctoat in 32 g Triethylenglycolmonomethylether in 2 Stunden zudosiert und 1,5 Stunden bei dieser Temperatur weiter auspolymerisiert. Da das Reaktionsgemisch im Laufe der Reaktion hochviskos wird 45 Minuten nach Polymerisationsbeginn innerhalb von 1,5 Stunden 250 g destilliertes Wasser zudosiert. Nach Ende der Reaktion wird mit 500 g destilliertem Wasser verdünnt. Die leicht gelbliche Polymerlösung weist einen K-Wert von 45 auf.

### Beispiel 6

Unter Verwendung von 72 g Alkylpolyethlenglykol mit einem mittleren Molekulargewicht von 3500 (Pluriol A 2000, BASF Aktiengesellschaft) wird analog Beispiel 5 die Polymerisation durchgeführt. Die erhaltene Polymerlösung weist einen K-Wert von 48 auf.

### Beispiel 7

Unter Verwendung von 103 g Polyethylenglykol mit einem mittleren Molekulargewicht von 20000 wird analog Beispiel 5 die Polymerisation durchgeführt. Die erhaltene Polymerlösung weist einen K-Wert von 53 auf.

### Beispiel 8

Unter Verwendung von 137 g Polyethylenglykol mit einem mittleren Molekulargewicht von 35000 wird analog Beispiel 5 die Polymerisation durchgeführt. Die erhaltene Polymerlösung weist einen K-Wert von 57 auf.

### Beispiel 9

Unter Verwendung von 103 g Polyethylenglykol mit einem mittleren Molekulargewicht von 20000 wird analog Beispiel 5 die Polymerisation durchgeführt. Die erhaltene Polymerlösung weist einen K-Wert von 55 auf.

### Beispiel 10

Unter Verwendung von 202 g Dimethicone copolyol (Belsil DMC 6031TM, Fa. Wacker Chemie GmbH) wird analog Beispiel 5 die Polymerisation durchgeführt. Die erhaltene Polymerlösung weist einen K-Wert von 47 auf.

### Beispiel 11

Unter Verwendung von 137 g ethoxiliertem Polyethylenimin (hergestellt aus 12,5 % Polyethylenimin mit einem mittlerem Molekulargewicht von 1400 und 87,5 % Ethylenoxid) wird analog Beispiel 5 die Polymerisation durchgeführt. Die erhaltene Polymerlösung weist einen K-Wert von 49 auf.

### Beispiel 12

In einem gerührten Reaktor mit Stickstoffzuführung, Rückflusskühler und Dosiervorrichtung werden 1000 g einer 21,4%igen Lösung von Polyvinylpyrrolidon mit einem K-Wert von 85,0 im schwachen Stickstoffstrom auf 80°C erhitzt. Innerhalb von zwei Stunden werden nun 91,7 g N-Vinylformamid und binnen 2,5 Stunden 1,83 g 2,2'-Azobis(2-amidinopropan)-dihydrochlorid, gelöst in 98,2 g Wasser, gleichmäßig zudosiert. Nach Beendigung des Monomerzulaufes wird der Reaktionsansatz mit 239 g Wasser verdünnt. Anschließend wird für 30 Minuten nachpolymerisiert, die Temperatur auf 85°C erhöht und unter Zugabe von 0,9 g 2,2'-Azobis(2-amidinopropan)-dihydrochlorid für eine weitere Stunde auspolymerisiert. Die erhaltene Polymerlösung besitzt einen Feststoffgehalt von 22,5 % und einen K-Wert von 85,1 (gemessen in 1%iger wässriger Lösung).

### Beispiel 13

In einem gerührten Reaktor mit Stickstoffzuführung, Rückflusskühler und Dosiervorrichtung werden 1000 g einer 21;0%igen Lösung von Polyvinylpyrrolidon mit einem K-Wert von 90, 339 g Wasser und 0,9 g Natriumdihydrogenphosphat im schwachen Stickstoffstrom auf 80°C erhitzt. Innerhalb von zwei Stunden werden nun 90 g N-Vinylformamid und binnen 2,5 Stunden 1,8 g 2,2'-Azobis(2-amidinopropan)-dihydrochlorid, gelöst in 98,2 g Wasser, gleichmäßig zudosiert. Nach Beendigung des Monomerzulaufes wird der Reaktionsansatz mit 300 g Wasser verdünnt. Anschließend wird für 30 Minuten nachpolymerisiert, dann die Temperatur auf 85°C erhöht und unter Zugabe von 0,5 g 2,2'-Azobis(2-amidinopropan)-dihydrochlorid für eine weitere Stunde auspolymerisiert. Die erhaltene Polymerlösung besitzt einen Feststoffgehalt von 16,1 % und einen K-Wert von 87,6 (gemessen in 1%iger wässriger Lösung).

### Beispiel 14

In einem gerührten Reaktor mit Stickstoffzuführung, Rückflusskühler und Dosiervorrichtung werden 428,6 g einer 21,0%igen Lösung von Polyvinylpyrrolidon mit einem K-Wert von 90, 690 g Wasser und 2,1 g Natriumdihydrogenphosphat im schwachen Stickstoffstrom auf 80°C erhitzt. Innerhalb von zwei Stunden werden nun 210 g N-Vinylformamid und binnen 2,5 Stunden 4,2 g 2,2'-Azobis(2-amidinopropan)-dihydrochlorid, gelöst in 97,8 g Wasser, gleichmäßig zudosiert. Nach Beendigung des Monomerzulaufes wird der Reaktionsansatz mit 200 g Wasser verdünnt. Anschließend wird für 30 Minuten nachpolymerisiert, dann die Temperatur auf 85°C erhöht und unter Zugabe von 0,8 g 2,2'-Azobis(2-amidinopropan)-dihydrochlorid für eine weitere Stunde auspolymerisiert. Die erhaltene Polymerlösung besitzt einen Feststoffgehalt von 18,4 % und einen K-Wert von 71,7 (gemessen in 1%iger wässriger Lösung).

### Beispiel 15

In einem gerührten Reaktor mit Stickstoffzuführung, Rückflusskühler und Dosiervorrichtung werden 214 g einer 21,0%igen Lösung von Polyvinylpyrrolidon mit einem K-Wert von 90, 428 g Wasser und 19,3 g N-Vinylformamid im schwachen Stickstoffstrom auf 80°C erhitzt. 0,39 g 2,2'-Azobis(2-amidinopropan)-dihydrochlorid werden nun auf einmal zugegeben und bei der Reaktionstemperatur für zwei Stunden polymerisiert. Anschließend wird die Temperatur auf 85°C erhöht und unter Zugabe von 0,18 g 2,2'-Azobis(2-amidinopropan)-dihydrochlorid für eine weitere Stunde auspolymerisiert. Die erhaltene Polymerlösung besitzt einen Feststoffgehalt von 9,7 % und einen K-Wert von 85 (gemessen in 1%iger wässriger Lösung).

### Beispiel 16

In einem gerührten Reaktor mit Stickstoffzuführung, Rückflusskühler und Dosiervorrichtung werden 231 g einer 30,3%igen Lösung von Polyvinylpyrrolidon mit einem K-Wert von 30, 405 g Wasser, 0,7 g Natriumdihydrogenphosphat und 30 g N-Vinylformamid im schwachen Stickstoffstrom auf 80°C erhitzt. An die Reaktionsapparatur wird nun soviel Vakuum angelegt, dass bei der Reaktionstemperatur der Ansatz leicht unter Rückfluss siedet. 0,6 g 2,2'-Azobis(2-amidinopropan)-dihydrochlorid werden nun auf einmal zugegeben und bei der Reaktionstemperatur für zwei Stunden polymerisiert. Anschließend wird die Temperatur auf 85°C erhöht und unter Zugabe von 0,3 g 2,2'-Azobis(2-amidinopropan)-dihydrochlorid für eine weitere Stunde auspolymerisiert. Die erhaltene Polymerlösung besitzt einen Feststoffgehalt von 15,3 % und einen K-Wert von 36,7 (gemessen in 1%iger wässriger Lösung).

### Beispiel 17

In einem gerührten Reaktor mit Stickstoffzuführung, Rückflusskühler und Dosiervorrichtung werden 99 g einer 30,3%igen Lösung von Polyvinylpyrrolidon mit einem K-Wert von 30, 498 g Wasser, 0,7 g Natriumdihydrogenphosphat und 70 g N-Vinylformamid im schwachen Stickstoffstrom auf 90°C erhitzt. 1,4 g 2,2'-Azobis-(2-amidinopropan)-dihydrochlorid werden nun auf einmal zugegeben und bei der Reaktionstemperatur für zwei Stunden polymerisiert. Anschließend wird die Temperatur auf 95°C erhöht und unter Zugabe von 0,7 g 2,2'-Azobis(2-amidinopropan)-dihydrochlorid für eine weitere Stunde auspolymerisiert. Die erhaltene Polymerlösung besitzt einen Feststoffgehalt von 13,8 % und einen K-Wert von 59,5 (gemessen in 1%iger wässriger Lösung).

### Beispiel 18

In einem gerührten Reaktor mit Stickstoffzuführung, Rückflusskühler und Dosiervorrichtung werden 115,8 g einer 21,6%igen Lösung von Polyvinylpyrrolidon mit einem K-Wert von 90, 484 g Wasser, 0,3 g Natriumdihydrogenphosphat und 25 g N-Vinylformamid im schwachen Stickstoffstrom auf 70°C erhitzt. An die Reaktionsapparatur wird nun soviel Vakuum angelegt, dass bei der Reaktionstemperatur der Ansatz leicht unter Rückfluss siedet. 0,5 g 2,2'-Azobis(2-amidinopropan)-dihydrochlorid werden nun auf einmal zugegeben und bei der Reaktionstemperatur für drei Stunden polymerisiert. Anschließend wird das Vakuum aufgehoben, die Temperatur auf 85°C erhöht und unter Zugabe von weiteren 0,25 g 2,2'-Azobis(2-amidinopropan)-dihydrochlorid eine weitere Stunde auspolymerisiert. Die erhaltene Polymerlösung besitzt einen Feststoffgehalt von 7,6 % und einen K-Wert von 74,9 (gemessen in 1%iger wässriger Lösung).

### Beispiel 19

In einem gerührten Reaktor mit Stickstoffzuführung, Rückflusskühler und Dosiervorrichtung werden 116,8 g einer 21,4%igen Lösung von Polyvinylpyrrolidon mit einem K-Wert von 85, 483 g Wasser, 0,3 g Natriumdihydrogenphosphat und 25 g N-Vinylformamid im schwachen Stickstoffstrom auf 70°C erhitzt. An die Reaktionsapparatur wird nun soviel Vakuum angelegt, dass bei der Reaktionstemperatur der Ansatz leicht unter Rückfluss siedet. 0,5 g 2,2'-Azobis(2-amidinopropan)-dihydrochlorid werden nun auf einmal zugegeben und bei der Reaktionstemperatur für drei Stunden polymerisiert. Anschließend wird das Vakuum aufgehoben, die Temperatur auf 85°C erhöht und unter Zugabe von weiteren 0,25 g 2,2`-Azobis(2-amidinopropan)-dihydrochlorid eine weitere Stunde auspolymerisiert. Die erhaltene Polymerlösung besitzt einen Feststoffgehalt von 7,8 % und einen K-Wert von 78 (gemessen in 1%iger wässriger Lösung).

### Beispiel 20

Analog zu Beispiel 17 wird die Polymerisation unter Verwendung eines Copolymerisats aus 70 Gew.-% Vinylpyrrolidon und 30 Gew.-% Vinylacetat mit einem K-Wert von 30 (Luviskol VA 73, BASF Aktiengesellschaft) als Pfropfgrundlage verwendet. Das erhaltene Polymer weist ein K-Wert von 55 auf.

### Beispiel 21

Analog zu Beispiel 17 wird die Polymerisation unter Verwendung eines Copolymerisats aus 60 Gew.-% Vinylpyrrolidon und 40 Gew.-% Vinylacetat mit einem K-Wert von 30 (Luviskol VA 64, BASF Aktiengesellschaft) als Pfropfgrundlage verwendet. Das erhaltene Polymer weist ein K-Wert von 53 auf.

### Beispiel 22

In einem gerührten Reaktor mit Stickstoffzuführung, Rückflusskühler und Dosiervorrichtung werden 165 g einer 30,3%igen Lösung von Polyvinylpyrrolidon mit einem K-Wert von 30, 451,5 g Wasser, 0,5 g Natriumdihydrogenphosphat und 50 g N-Vinylformamid im schwachen Stickstoffstrom auf 90°C erhitzt. 1,0 g 2,2'-Azobis-(2-amidinopropan)-dihydrochlorid werden nun auf einmal zugegeben und bei der Reaktionstemperatur für zwei Stunden polymerisiert. Anschließend wird die Temperatur auf 95°C erhöht und unter Zugabe von 0,5 g 2,2'-Azobis(2-amidinopropan)-dihydrochlorid für eine weitere Stunde auspolymerisiert. Die erhaltene Polymerlösung besitzt einen Feststoffgehalt von 14,4 % und einen K-Wert von 38,6 (gemessen in 1%iger wässriger Lösung).

### Beispiel 23: Verseifung von Beispiel 22

450 g des Polymeren aus Beispiel 22 werden auf 80°C erhitzt. Innerhalb einer Stunde werden 52 g 50%ige Natronlauge gleichmäßig zugetropft. Anschließend wird zwei Stunden nachgerührt, abgekühlt und mit 62 g konzentrierter Salzsäure auf pH 7 gestellt. Der Hydrolysegrad beträgt 100 %.

### Beispiel 24: Verseifung von Beispiel 22

450 g des Polymeren aus Beispiel 22 werden auf 80°C erhitzt. Innerhalb einer Stunde werden 26 g 50%ige Natronlauge gleichmäßig zugetropft. Anschließend wird zwei Stunden nachgerührt, abgekühlt und mit 31 g konzentrierter Salzsäure auf pH 7 gestellt. Der Hydrolysegrad beträgt 50 %.

### Beispiel 25

In einem gerührten Reaktor mit Stickstoffzuführung, Rückflusskühler und Dosiervorrichtung werden 150 g einer 20%igen Lösung teilverseiftem Polyvinylalkohol mit einem mittlerem Molekulargewicht von 31000 (Mowiol 4-88, Fa. Clariant), 498 g Wasser, 0,7 g Natriumdihydrogenphosphat und 70 g N-Vinylformamid.im schwachen Stickstoffstrom auf 90°C erhitzt. 1,4 g 2,2'-Azobis-(2-amidinopropan)-dihydrochlorid werden nun auf einmal zugegeben und bei der Reaktionstemperatur für zwei Stunden polymerisiert. Anschließend wird die Temperatur auf 95°C erhöht und unter Zugabe von 0,7 g 2,2'-Azobis(2-amidinopropan)-dihydrochlorid für eine weitere Stunde auspolymerisiert. Das erhaltene Polymer wies einen K-Wert von 45,3 (gemessen in 1%iger wässriger Lösung).

### Beispiel 26

In einem gerührten Reaktor mit Stickstoffzuführung, Rückflusskühler und Dosiervorrichtung werden 150 g einer 20%igen Lösung teilverseiftem Polyvinylalkohol mit einem mittlerem Molekulargewicht von 67000 (Mowiol 8-88, Fa. Clariant), 498 g Wasser, 0,7 g Natriumdihydrogenphosphat und 70 g N-Vinylformamid im schwachen Stickstoffstrom auf 90°C erhitzt. 1,4 g 2,2'-Azobis-(2-amidinopropan)-dihydrochlorid werden nun auf einmal zugegeben und bei der Reaktionstemperatur für zwei Stunden polymerisiert. Anschließend wird die Temperatur auf 95°C erhöht und unter Zugabe von 0,7 g 2,2'-Azobis(2-amidinopropan)-dihydrochlorid für eine weitere Stunde auspolymerisiert. Das erhaltene Polymer wies einen K-Wert von 65,3 (gemessen in 1%iger wässriger Lösung).

### Beispiel 27

In einem gerührten Reaktor mit Stickstoffzuführung, Rückflusskühler und Dosiervorrichtung werden 200 g einer 15%igen Lösung vollverseiftem Polyvinylalkohol mit einem mittlerem Molekulargewicht von 61000 (Mowiol 10-98, Fa. Clariant), 498 g Wasser, 0,7 g Natriumdihydrogenphosphat und 70 g N-Vinylformamid im schwachen Stickstoffstrom auf 90°C erhitzt. 1,4 g 2,2'-Azobis(2-amidinopropan)-dihydrochlorid werden nun auf einmal zugegeben und bei der Reaktionstemperatur für zwei Stunden polymerisiert. Anschließend wird die Temperatur auf 95°C erhöht und unter Zugabe von 0,7 g 2,2'-Azobis(2-amidinopropan)-dihydrochlorid für eine weitere Stunde auspolymerisiert. Das erhaltene Polymer wies einen K-Wert von 68,4 (gemessen in 1%iger wässriger Lösung).

### Beispiel 28: Verseifung von Beispiel 27

450 g des Polymeren aus Beispiel 22 werden auf 80°C erhitzt. Innerhalb einer Stunde werden 52 g 50%ige Natronlauge gleichmäßig zugetropft. Anschließend wird zwei Stunden nachgerührt, abgekühlt und mit 62 g konzentrierter Salzsäure auf pH 7 gestellt. Der Hydrolysegrad beträgt 100 %.

### Beispiel 29

In einem gerührten Reaktor mit Stickstoffzuführung, Rückflusskühler und Dosiervorrichtung werden 1010 g destilliertes Wasser, 2,4 g primäres Natriumphosphat und 97,1 g Kartoffelstärke (82,4%ig) unter einem leichten Stickstoffstrom auf 70°C aufgeheizt. Bei dieser Temperatur werden 120 g N-Vinylformamid über 2 Stunden und 0,98 g 2,2'-Azobies-(2-methylproionamidin)dihydrochlorid in 97,6 g destilliertem Wasser in 3 Stunden zugegeben. Die Reaktionslösung wird für weitere 3 Stunden bei 70°C gerührt. Die entstehende weiße Dispersion hat einen Festgehalt von 15,0 %.

### Beispiel 30

400 g der Dispersion aus Beispiel 24 (Gewichtsverhältnis N-Vinylformamid zu Kartoffelstärke = 60:40) werden in oben beschriebenen Reaktor mit 53,0 g 38%iger Salzsäure tropfenweise innerhalb von 5 Minuten zugegeben. Im Anschluss wird auf 70°C erhitzt für 8 Stunden. Eine 90%ige Hydrolyse des Polymers wird erreicht.

### Beispiel 31

In einem gerührten Reaktor mit Stickstoffzuführung, Rückflusskühler und Dosiervorrichtung werden 1993 g destilliertes Wasser, 3,6 g primäres Natriumphosphat und 40 g Maltodextrin unter einem leichten Stickstoffstrom auf 70°C erhitzt. Bei dieser Temperatur werden 160 g N-Vinylformamid über 3 Stunden und 0,98 g 2,2'-Azobis-(2-methylpropionamidin)dihydrochlorid in 95 g destilliertem Wasser in 4 Stunden zugegeben. Die Reaktionslösung wird für weitere 2 Stunden bei 70°C gerührt. Die leicht trübe Lösung hat einen Festgehalt von 14,6 % und einen K-Wert von 60.

### Beispiel 32

In einem gerührten Reaktor mit Stickstoffzuführung, Rückflusskühler und Dosiervorrichtung werden 1996 g destilliertes Wasser, 1,4 g primäres Natriumphosphat und 78,1 g Glycosesirup (76,8%ig) unter einem leichten Stickstoffstrom auf 70°C erhitzt. Bei dieser Temperatur werden 142 g N-Vinylformamid über 2 Stunden und 0,7 g 2,2'-Azobies-(2-methylproionamidin)dihydrochlorid in 95 g destilliertem Wasser in 3 Stunden zugegeben. Die Reaktionslösung wird für weitere 3 Stunden bei 70°C gerührt. Die klare, farblose, viskose Lösung hat einen Festgehalt von 15,7 % und einen K-Wert von 71,1.

### Beispiel 33

| | | |
|---|---|---|
| Zulauf 1 | Vinylpyrrolidon | 140 g |
| | Vinylformamid | 40 g |
| Zulauf 2 | Ethanol | 60 g |
| Wako V 59 | 2,2'-Azobis(2-methylbutyronitril) | 0.5 g |
| Zulauf 3 | Ethanol | 12 g |
| | Wako V 59 | 1.5 g |
| Vorlage | Ethanol | 30 g |
| | Wasser dest. | 120 g |
| | Mowiol 4/88 (20%ig) | 100 g |
| Zulauf 1 | | 18 g |
| Zulauf 2 | | 6 g |

In einem gerührten Reaktor mit Stickstoffzuführung, Rückflusskühler und Dosierungsvorrichtung werden 100 g 20%igen Lösung eines teilverseiften Polyvinylalkohol Mowiol 4 bis 88 (Fa. Clariant), 18 g von Zulauf 1,6 g von Zulauf 2 in 30 g Ethanol und 120 g Wasser vorgelegt im schwachen N2-Strom auf ca. 80°C erhitzt. Der restliche Zulauf 1 in 3 h und Zulauf 2 in 4 h zudosiert und polymerisiert.
Die Polymerlösung wird noch 1 h bei 80°C unter Rühren gehalten. Der Zulauf 3 wird in ca. 30 min. bei einer Temperatur von ca. 80°C zugegeben und das Produkt wird weiter noch 3 h nachpolymerisiert. Das erhaltene Polymer (im folgenden als 33d bezeichnet) wies einen K-Wert von 51.4 (gemessen in 1%iger N-Methylpyrrolidon.

Nach dieser allg. Vorschrift wurden folgende Pfropfcopolymer hergestellt:

| Beispiel Nr. | Vinylpyrrolidon (in Gew.-%) | Vinylformamid (in Gew.-%) | Polyvinylalkohol (in Gew.-%) | K-Wert (1 % in NMP) |
|---|---|---|---|---|
| 33 a | -- | 80 | 20 | ungelöst nicht bestimmbar |
| 33 b | 50 | 30 | 20 | 58.9 |
| 33 c | 60 | 20 | 20 | 57.2 |
| 33 d | 70 | 20 | 10 | 51.4 |
| 33 e | 50 | 20 | 30 | 49.8 |

Alle Produkte werden bevorzugt als Haarfestigerpolymere verwendet. Sie sind mit Verdicker auf Basis von Polyacrylsäure, z.B. Carbopol 940 ( Fa. Goodrich) sehr gut verträglich und lassen sich mit etwa 0.25 bis 2 Gew.-% Carbopol 940 in Wasser gute Gele formulieren.

### Anwendungsbeispiele

### Beispiel 1: Formulierung Aerosolhaarschaum:

| | |
|---|---|
| 2,00 % | Copolymer aus Beispiel 1 |
| 2,00 % | Luviquat Mono LS (Coco trimonium methyl sulfat) |
| 67,7 % | Wasser |
| 10,0 | Propan/Butan 3,5 bar (20°C) |
| q.s. | Parfümöl |

### Beispiel 2 (Vergleichsbeispiel):

| | |
|---|---|
| 2,00 % | Polymergehalt Luviquat Hold (Polquaternium-46) |
| 2,00 % | Luviquat Mono LS (Coco trimonium methyl sulfat) |
| 67,7 % | Wasser |
| 10,0 | Propan/Butan 3,5 bar (20°C) |
| q.s. | Parfümöl |

### Beispiel 3: Aerosolhaarschaum:

| | | INCI |
|---|---|---|
| 4,00 % | Copolymer aus Beispiel 17 | |
| 0,20 % | Cremophor A 25 | Ceteareth-25 |
| 1,00 % | Luviquat Mono CP | Hydroxyethyl cetyldimonium phosphate |
| 5,00 % | Ethanol | |
| 1,00 % | Panthenol | |
| 10,0 | Propan/Butan 3,5 bar (20°C) | |
| q.s. | Parfümöl | |
| ad 100 % | Wasser | |

### Beispiel 4: Pumpschaum:

| | | INCI |
|---|---|---|
| 2,00 % | Copolymer aus Beispiel 26 | |
| 2,00 % | Luviflex Soft (Polymergehalt) | |
| 1,20 % | 2-Amino-2-methyl-1-propanol | |
| 0,20 % | Cremophor A 25 | |
| 0,10 % | Uvinul P 25 | PEG-25 PABA |
| q.s. | Konservierungsmittel | |
| q.s. | Parfümöl | |
| ad 100 % | Wasser | |

### Beispiel 5 Pumpspray

| | | INCI |
|---|---|---|
| 4,00 % | Copolymer aus Beispiel 32 | |
| 1,00 | % Panthenol | |
| 0,10 | % Uvinul MS 40 | Benzophenone-4 |
| q.s. | Konservierungsmittel | |
| q.s. | Parfümöl | |
| ad 100 % | Wasser | |

### Beispiel 6: Pumpspray:

| | | INCI |
|---|---|---|
| 4,00 | % Copolymer aus Beispiel 22 | |
| 1,00 | % Panthenol | |
| 0,10 % | Uvinul M 40 | Benzophenone-3 |
| q.s. | Konservierungsmittel | |
| q.s. | Parfümöl | |
| ad 100 % | Ethanol | |

### Beispiel 7: Haarspray:

| | | INCI |
|---|---|---|
| 5,00 % | Copolymer aus Beispiel 10 | |
| 0,10 % | Siliconöl Dow Corning DC 190 | Dimethicone Copolyol |
| 35,00 % | Dimethylether | |
| 5,00 % | n-Pentan | |
| ad 100 % | Ethanol | |
| q.s. | Parfümöl | |

### Beispiel 8: Haarspray VOC 55 %:

| | | INCI |
|---|---|---|
| 3,00 % | Copolymer aus Beispiel 4 | |
| 7,00 % | Luviset P.U.R. | Polyurethane-1 |
| 40,00 % | Dimethylether | |
| 15,00 % | Ethanol | |
| q.s. | Parfümöl | |
| ad 100 % | Wasser | |

### Beispiel 9: Haargel:

| | | INCI |
|---|---|---|
| 0,50 % | Carbopol 980 | Cabomer |
| 3,00 % | Copolymer aus Beispiel 33b | |
| 0,10 % | Phythantriol | |
| 0,50 % | Panthenol | |
| q.s. | Parfümöl | |
| q.s | Konservierungsmittel | |
| ad 100 | % Wasser | |

### Beispiel 10: Haarshampoo bzw. Duschgel

| | | INCI |
|---|---|---|
| 0,50 % | Copolymer aus Beispiel 27 | |
| 40,00 % | Texapon NSO | Sodium Laureth Sulfate |
| 5,00 % | Tego Betain L 7 | Cocamidopropyl Betaine |
| 5,00 % | Plantacare 2000 | Decyl Glucoside |
| 1,00 % | Propylenglycol | |
| q.s. | Citronensäure | |
| q.s. | Konservierungsmittel | |
| 1,00 % | Natriumchlorid | |
| ad 100 % | Wasser | |

### Anwendungsbeispiel 11: Hautcreme

Gemäß folgender Rezeptur wurde zunächst eine erfindungsgemäße Wasser/Öl-Cremeemulsion (Hautcreme A) hergestellt:

| Zusatz | | Gew.-% |
|---|---|---|
| Cremophor A 6 | Ceteareth-6 und Stearylalkohol | 2,0 |
| Chremophor A 25 | Ceteareth-25 | 2,0 |
| Lanette O | Cetearylalkohol | 2,0 |
| Imwitor 960 K | Glycerylstearat SE | 3,0 |
| Paraffinöl | | 5,0 |
| Jojobaöl | | 4,0 |
| Luvitol EHO | Cetearyloctanoat | 3,0 |
| ABIL 350 | Dimethicone | 1,0 |
| Amerchol L 101 | Mineralöl und Lanolinalkohol | 3,0 |
| Veegum Ultra | Magnesiumaluminiumsilikat | 0,5 |
| 1,2-Propylenglykol | Propylenglykol | 5,0 |
| Abiol | Imidazolindinyl-Harnstoff | 0,3 |
| Phenoxyethanol | | 0,5 |
| D-Panthenol USP | | 1,0 |
| Polymer (Herstellungsbeispiel 28) | | 0,5 |
| Wasser | | ad 100 |

In gleicher Weise wurden zwei Vergleichscremes hergestellt, und zwar:

### Hautcreme B (ohne Polymerzusatz)

Mit diesen Hautcremes A und B wurden die folgenden Vergleichstests 1 und 2 zur Beurteilung des Hautgefühls durchgeführt.

100 ∝1 der Emulsion wurden gleichmäßig auf dem Handrücken verteilt und das Hautgefühl subjektiv nach 30 Minuten Einwirkzeit getestet. Es wurden jeweils zwei Emulsionen (rechte-linke Hand) miteinander verglichen. Der Test wurde von je 10 Probanden durchgeführt.

### Notenskala:

2 (deutlich zarter als Vergleichscreme)
1 (etwas zarter als Vergleichscreme)
0 (gleich)
-1 (etwas rauher als Vergleichscreme)
-2 (deutlich rauher als Vergleichscreme)

Ergebnis von Vergleichstest 1 (Vergleich der Hautcremes A und Vergleichs creme B):

| Note | Anzahl der Probanden |
|---|---|
| 2 | 5 |
| 1 | 4 |
| 0 | 1 |
| -1 | - |
| -2 | - |

### Anwendungsbeispiel 12: Duschgel

Gemäß folgender Rezeptur wurde zunächst eine erfindungsgemäße Duschcrel-Formulierung (Duschgel A) hercrestellt:

| Zusatz | | Gew.-% |
|---|---|---|
| Texapon NSO | Natriumlaurethsulfat | 40,0 |
| Tego Betain L7 | Cocamidopropylbetain | 5,0 |
| Plantacare 2000 | Decylglucosid | 5,0 |
| Parfüm | | 0,2 |
| Polymer gemäß Herstellungsbeispiel 30 | | 0,2 |
| Euxyl K 100 | Benzylalkohol, Methylchlorisothiazolinon, Methylisothiazolinon | 0,1 |
| D-Panthenol USP | | 0,5 |
| Citronensäure (pH 6-7) | | q.s. |
| NaCl | | 2,0 |
| Wasser | | ad 100 |

In gleicher Weise wurden drei Vergleichs-Duschgele hergestellt und zwar:
- Duschgel B:: (erfindungsgemäßes Copolymer ersetzt durch gleiche Menge kationisch modifizierter Hydroxyethylcellulose)
- Duschgel C:: (ohne Polymerzusatz)

Mit den Duschgelen A, B, und C wurde der folgende Vergleichs-test 3 zur Bestimmung der Schaumcremigkeit durchgeführt:

Je 2,0 g der oben genannten Formulierung wurde auf die linke Handinnenfläche gegeben, mit Leitungswasser angeschäumt und nach 1 Minute Reiben zwischen beiden Händen das Schaumgefühl in den Handinnenflächen beurteilt:
Note 1: sehr cremig/sahnig
Note 2: cremig/sahnig
Note 3: stumpf/gehaltlos

Ergebnis von Vergleichstest 3 (Mittelwert der Benotung durch 10 Probanden):

| Duschgel | Mittelwert von 10 Probanden |
|---|---|
| A | 1,3 |
| B | 2,1 |
| C | 2,8 |

### Anwendungsbeispiel 13: Feuchthalteformulierung

### Formulierung A

| Zusatz | | | Gew.-% |
|---|---|---|---|
| a) | Cremophor A6 | Ceteareth-6 und Stearylalkohol | 2,0 |
| | Cremophor A25 | Ceteareth-25 | 2,0 |
| | Paraffinöl (dickflüssig) | | 10 |
| | Lannette O | Cetearylalkohol | 2,0 |
| | Stearinsäure | | 3,0 |
| | Nip-Nip | Methylparaben/Propylparaben 70:30 | 0,5 |
| | Abiol | Imidazoldinyl-Harnstoff | 0,5 |
| b) | Polymer (Herstellungsbeispiel 3) | | 3,0 |
| | Wasser | | ad 100,0 |

Beide Phasen wurden auf 80°C erhitzt, Phase a) wurde in b) eingerührt, homogenisiert und kaltgerührt und anschließend mit 10%iger wässriger NaOH-Lösung auf pH 6 eingestellt.

In gleicher Weise wurde eine Vergleichscreme (Formulierung B) ohne Polymerzusatz hergestellt.

Mit den Formulierungen A und B wurde ein Probandentest an 8 Probanden durchgeführt. Dazu wurden die Formulierungen jeweils auf den Unterarm der Probanden in einer Menge von 2 mg/cm² aufgetragen. Nach 30 min wurde der Feuchtigkeitsgehalt der Haut mit einem Corneometer CM 825 (Fa. Khazaka & Courage) bestimmt. Nach Applikation von Formulierung A wurde ein durchschnittlicher Wert von 45 Corneometereinheiten gemessen, mit Formulierung B ein durchschnittlicher Wert von 35.

### Anwendungsbeispiel 14: O/W Creme zur Hautfeuchthaltung

| Zusatz | Gew.-% |
|---|---|
| Glycerinmonostearat | 2,0 |
| Cetylalkohol | 3,0 |
| Paraffinöl, subliquidum | 15,0 |
| Vaseline | 3,0 |
| Caprylcaprinsäuretriglycerid | 4,0 |
| Octyldodecanol | 2,0 |
| Hydriertes Kokosfett | 2,0 |
| Cetylphosphat | 0,4 |
| Polymer (Herstellungsbeispiel 33) | 3,0 |
| Glycerin | 3,0 |
| Natriumhydroxid | q.s. |
| Parfümöl | q.s. |
| Konservierungsmittel | q.s. |
| Wasser | ad 100 |

### Anwendungsbeispiel 15: O/W-Lotion

| Zusatz | Gew.-% |
|---|---|
| Stearinsäure | 1,5 |
| Sorbitanmonostearat | 1,0 |
| Sorbitanmonooleat | 1,0 |
| Paraffinöl, subliquidum | 7,0 |
| Cetylalkohol | 1,0 |
| Polydimethylsiloxan | 1,5 |
| Glycerin | 3,0 |
| Polymer (Herstellungsbeispiel 30) | 0,5 |
| Parfümöl | q.s. |
| Konservierungsmittel | q.s. |
| Wasser | ad 100 |

### Anwendungsbeispiel 16: W/O-Creme

| Zusatz | Gew.-% |
|---|---|
| PEG-7-hydriertes Ricinusöl | 4,0 |
| Wollwachsalkohol | 1,5 |
| Bienenwachs | 3,0 |
| Triglycerid, flüssig | 5,0 |
| Vaseline | 9,0 |
| Ozokerite | 4,0 |
| Paraffinöl, subliquidum | 4,0 |
| Glycerin | 2,0 |
| Polymer (Herstellungsbeispiel 29) | 2,0 |
| Magnesiumsulfat*7H₂0 | 0,7 |
| Parfümöl | q.s. |
| Konservierungsmittel | q.s. |
| Wasser | ad 100 |

### Anwendungsbeispiel 17: Hydrogel zur Hautpflege

| Zusatz | Gew.-% |
|---|---|
| Polymer (Herstellungsbeispiel 10) | 3,0 |
| Sorbit | 2,0 |
| Glycerin | 3,0 |
| Polyethylenglycol 400 | 5,0 |
| Ethanol | 1,0 |
| Parfümöl | q.s. |
| Konservierungsmittel | q.s. |
| Wasser | ad 100 |

### Anwendungsbeispiel 18: Hydrodispersionsgel

| Zusatz | Gew.-% |
|---|---|
| Polymer (Herstellungsbeispiel 26) | 3,0 |
| Sorbit | 2,0 |
| Glycerin | 3,0 |
| Polyethylenglycol 400 | 5,0 |
| Triglycerid, flüssig | 2,0 |
| Ethanol | 1,0 |
| Parfümöl | q.s. |
| Konservierungsmittel | q.s. |
| Wasser | ad 100 |

### Anwendungsbeispiel 19: Flüssige Seife

| Zusatz | Gew.-% |
|---|---|
| Kokosfettsäure, Kaliumsalz | 15 |
| Kaliumoleat | 3 |
| Glycerin | 5 |
| Polymer (Herstellungsbeispiel 28) | 2 |
| Glycerinstearat | 1 |
| Ethylenglycoldistearat | 2 |
| Spezifische Zusätze, Komplexierungsmittel, Duftstoffe | q.s. |
| Wasser | ad 100 |

### Anwendungsbeispiel 20: Body Care Cream

| Zusatz | | Gew.-% |
|---|---|---|
| Cremophor A6 | Ceteareth-6 und Stearylalkohol | 2,0% |
| Cremophor A 25 | Ceteareth-25 | 2,0% |
| Grape (Vitis Vinifera) | Seed oil | 6,0% |
| Glycerylstearat SE | | 3,0% |
| Cetearylalkohol | | 2,0% |
| Dimethicon | | 0,5% |
| Luvitol EHO | Cetearyloctanoat | 8,0% |
| Oxynex 2004 | Propylenglycol, BHT, Ascorbylpalmitat, Glycerylstearat, Citronensäure | 0,1% |
| Konservierungsmittel | | q.s. |
| 1,2-Propylenglykol USP | | 3,0% |
| Glycerin | | 2,0% |
| EDTA BD | | 0,1% |
| D-Panthenol USP | | 1,0% |
| Wasser | | ad 100 |
| Polymer (Herstellungsbeispiel 7) | | 1,5% |
| Tocopherylacetat | | 0,5% |

Die Formulierung wies einen pH-Wert von 6,8 auf. Die Viskosität (Brookfield

In den folgenden Anwendungsbeispielen sind alle Mengenangaben in Gew.-%.

### Anwendungsbeispiel 21: Flüssiges Makeup

| A | |
|---|---|
| 1,70 | Glyceryl Stearate |
| 1,70 | Cetyl Alcohol |
| 1,70 | Ceteareth-6 |
| 1,70 | Ceteareth-25 |
| 5,20 | Caprylic/Capric Triglyceride |
| 5,20 | Mineral Oil |

| B | |
|---|---|
| q.s. | Konservierungsmittel |
| 4,30 | Propylene Glycol |
| 2,50 | Polymerisat gemäß Herstellbeispiel 11 |
| 59,50 | dest. Wasser |

| C | |
|---|---|
| q.s. | Parfumöl |

| D | |
|---|---|
| 2,00 | Iron Oxides |
| 12,00 | Titanium Dioxide |

### Herstellung:

Phase A und Phase B getrennt voneinunder auf 80°C erwärmen. Dann Phase B in Phase A mit einem Rührer mischen. Alles auf 40°C abkühlen lassen und Phase C und Phase D zugeben. Wiederholt homogenisieren.

### Anwendungsbeispiel 22: Ölfreies Makeup

| A | |
|---|---|
| 0,35 | Veegum |
| 5,00 | Butylene Glykol |
| 0,15 | Xanthan Gum |

| B | |
|---|---|
| 53,0 | dest. Wasser |
| q.s. | Konservierungsmittel |
| 0,2 | Polysorbate-20 |
| 1, 6 | Tetrahydroxypropyl Ethylenediamine |

| C | |
|---|---|
| 1,0 | Silica |
| 2,0 | Nylone-12 |
| 4,15 | Mica |
| 6,0 | Titanium Dioxide |
| 1,85 | Iron Oxides |

| D | |
|---|---|
| 4,0 | Stearic Acid |
| 1,5 | Glyceryl Stearate |
| 7,0 | Benzyl Laurate |
| 5,0 | Isoeicosane |
| q.s. | Konservierungsmittel |

| E | |
|---|---|
| 1,0 | dest. Wasser |
| 0,5 | Panthenol |
| 0,1 | Imidazolidinyl Urea |
| 5,0 | Polymerisat gemäß Herstellbeispiel 6 |

### Herstellung:

Phase A mit Butylene Glykol benetzen, in Phase B hineingeben und gut mischen. Phase AB auf 75°C erwärmen. Phase C Einsatzstoffe pulverisieren, in Phase AB hineingeben und gut homogenisieren. Einsatzstoffe von Phase D mischen, auf 80°C erwärmen und zu Phase ABC geben. Einige Zeit mischen, bis alles homogen ist. Alles in ein Gefäß mit Propellermischer übertragen. Einsatzstoffe von Phase E mischen, in Phase ABCD hineingeben und gut vermischen.

### Anwendungsbeispiel 23 Eyeliner

| A | |
|---|---|
| 40,6 | dest, Wasser |
| 0,2 | Disodium EDTA |
| q.s. | Konservierungsmittel |

| B | |
|---|---|
| 0,6 | Xanthan Gum |
| 0,4 | Veegum |
| 3,0 | Butylene Glycol |
| 0,2 | Polysorbate-20 |

| C | |
|---|---|
| 15,0 | Iron oxide / A1 Powder / Silica (z.B. Sicopearl Fantastico Gold ™ von BASF) |

| D | |
|---|---|
| 10,0 | Dest. Wasser |
| 30,0 | Polymerisat gemäß Herstellbeispiel 9 |

### Herstellung:

Phase B vormischen. Mit einem Propellermischer Phase B in Phase hineinmischen, wobei man den Verdicker quellen lässt. Phase C mit Phase D benetzen, alles in Phases AB zugeben und gut mischen.

### Anwendungsbeisipiel 24 Schimmerndes Gel

| A | |
|---|---|
| 32,6 | Dest. Wasser |
| 0,1 | Disodium EDTA |
| 25,0 | Carbomer (2%ige wässrige Lösung) |
| 0,3 | Konservierungsmittel |

| B | |
|---|---|
| 0,5 | Dest. Wasser |
| 0,5 | Triethanolamine |

| C | |
|---|---|
| 10,0 | Dest. Wasser |
| 9,0 | Polymerisat gemäß Herstellbeispiel 31 |
| 1,0 | Polyquaternium-46 |
| 5,0 | Iron Oxide |

| D | |
|---|---|
| 15,0 | Dest. Wasser |
| 1,0 | D-Panthenol 50 P (Panthenol und Propylene Glycol) |

### Herstellung:

Mit einem Propellermischer die Einsatzstoffe von Phase A in der angegebenen Reihenfolge gut mischen. Dann Phase B in Phase A hineingeben. Langsam rühren bis alles homogen ist. Phase C gut homogenisieren, bis die Pigmente gut verteilt sind. Phase C und Phase D zu Phase AB geben und gut mischen.

### Anwendungsbeispiel 25: Wasserfester Mascara

| A | |
|---|---|
| 46,7 | Dest. Wasser |
| 3,0 | Lutrol E 400 (PEG-8) |
| 0,5 | Xanthan Gum |
| q.s. | Konservierungsmittel |
| 0,1 | Imidazolidinyl Urea |
| 1,3 | Tetrahydroxypropyl Ethylenediamine |

| B | |
|---|---|
| 8 , 0 | Carnauba Wax |
| 4,0 | Beeswax |
| 4,0 | Isoeicosane |
| 4,0 | Polyisobutene |
| 5,0 | Stearic Acid |
| 1,0 | Glyceryl Stearate |
| q.s. | Konservierungsmittel |
| 2,0 | Benzyl Laurate |

| C | |
|---|---|
| 10,0 | Iron oxide / A1 Powder / Silica (z.B. Sicopearl Fantasticc Gold ™ von BASF) |

| E | |
|---|---|
| 8,0 | Polyurethane-1 |
| 2,0 | Polymerisat gemäß Herstellbeispiel 38 |

### Herstellung:

Phase A und Phase B getrennt voneinander auf 85ºC erwärmen. Temperatur halten und Phase C zu Phase A geben und homogenisieren, bis die Pigmente gleichmäßig verteilt sind. Phase B zu Phases AC geben und für 2-3 Minuten homogenisieren. Dann Phase E zugeben und langsam rühren. Alles auf Raumtemperatur abkühlen lassen.

### Anwendungsbeispiel 26 Sonnenschutz-Gel

| Phase A | |
|---|---|
| 1,00 | PEG-40 Hydrogenated Castor Oil |
| 8,00 | Octyl Methoxycinnamate (Uvinul MC 80™ von BASF) |
| 5,00 | Octocrylene (Uvinul N 539 ™ von BASF) |
| 0,80 | Octyl Triazone (Uvinul T 150™ von BASF) |
| 2,00 | Butyl Methoxydibenzoylmethane (Uvinul BMBM™ von BASF) |
| 2,00 | Tocopheryl Acetate |
| q.s. | Parfümöl |

| Phase B | |
|---|---|
| 2,50 | Polymerisat gemäß Herstellbeispiel 26 |
| 0,30 | Acrylates/C10-30 Alkyl Acrylate Crosspolymer |
| 0,20 | Carbomer |
| 5,00 | Glycerin |
| 0,20 | Disodium EDTA |
| q.s. | Konservierungsmittel |
| 72,80 | dest. Wasser |

| Phase C | |
|---|---|
| 0,20 | Sodium Hydroxide |

### Herstellung:

Die Komponenten der Phase A mischen. Phase B quellen lassen und unter Homogenisieren in Phase A einrühren. Mit Phase C neutralisieren und erneut homogenisieren.

### Anwendungsbeispie 27 Sonnenschutzemulsion mit TiO₂ und ZnO₂

| Phase A | |
|---|---|
| 6,00 | PEG-7 Hydrogenated Castor Oil |
| 2,00 | PEG-45/Dodecyl Glycol Copolymer |
| 3,00 | Isopropyl Myristate |
| 8,00 | Jojoba (Buxus Chinensis) Oil |
| 4,00 | Octyl Methoxycinnamate (Uvinul MC 80) |
| 2,00 | 4-Methylbenzylidene Camphor (Uvinul MBC 95) |
| 3,00 | Titanium Dioxide, Dimethicone |
| 1,00 | Dimethicone |
| 5,00 | Zinc Oxide, Dimethicone |

| Phase B | |
|---|---|
| 2,00 | Polymerisat gemäß Herstellbeispiel 24 |
| 0,20 | Disodium EDTA |
| 5,00 | Glycerin |
| q.s. | Konservierungsmittel |
| 58,80 | dest. Wasser |

| Phase C | |
|---|---|
| q.s. | Parfümöl |

### Herstellung:

Die Phasen A und B getrennt auf ca. 85°C erwärmen. Phase B in Phase A einrühren und homogenisieren. Abkühlen auf ca. 40°C, Phase C hinzugeben und nochmals kurz homogenisieren.

### Anwendungsbeispiel 28 Sun Protection Lotion

| Phase A | |
|---|---|
| 6,00 | Octyl Methoxycinnamate (Uvinul MC 80 ™ von BASF) |
| 2,50 | 4-Methylbenzylidene Camphor (Uvinul MBC 95 ™ von BASF) |
| 1,00 | Octyl Triazone (Uvinul T 150 ™ von BASF) |
| 2,00 | Butyl Methoxydibenzoylmethane (Uvinul BMBM ™ von BASF) |
| 2,00 | PVP/Hexadecene Copolymer |
| 5,00 | PPG-3 Myristyl Ether |
| 0,50 | Dimethicone |
| 0,10 | BHT, Ascorbyl Palmitate, Citric Acid, Glyceryl Stearate, Propylene Glycol |
| 2,00 | Cetyl Alcohol |
| 2,00 | Potassium Cetyl Phosphate |

| Phase B | |
|---|---|
| 2,50 | Polymerisat gemäß Herstellbeispiel 25 |
| 5,00 | Propylene Glycol |
| 0,20 | Disodium EDTA |
| q.s. | Konservierungsmittel |
| 63,92 | dest. Wasser |

| Phase C | |
|---|---|
| 5,00 | Mineral Oil |
| 0,20 | Carbomer |

| Phase D | |
|---|---|
| 0,08 | Sodium Hydroxide |

| Phase E | |
|---|---|
| q.s. | Parfümöl |

### Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

### Abwendungsbeispiel 29: Abziehbare Gesichtsmaske

| Phase A | |
|---|---|
| 57,10 | dest. Wasser |
| 6,00 | Polyvinyl Alcohol |
| 5,00 | Propylene Glycol |

| Phase B | |
|---|---|
| 20,00 | Alcohol |
| 4,00 | PEG-32 |
| q. s | Parfümöl |

| Phase C | |
|---|---|
| 5,00 | Polyquaternium-44 |
| 2,70 | Polymerisat gemäß Herstellbeispiel 8 |
| 0,20 | Allantoin |

### Herstellung:

Phase A auf mind. 90°C erwärmen und rühren bis gelöst. Phase B bei 50°C lösen und in Phase A einrühren. Bei ca. 35°C den Ethanolverlust ausgleichen. Phase C zugeben und unterrühren.

### Anwendungsbeispiel 30: Gesichtsmaske

| Phase A | |
|---|---|
| 3,00 | Ceteareth-6 |
| 1,50 | Ceteareth-25 |
| 5,00 | Cetearyl Alcohol |
| 6,00 | Cetearyl Octanoate |
| 6,00 | Mineral Oil |
| 0,20 | Bisabolol |
| 3,00 | Glyceryl Stearate |

| Phase B | |
|---|---|
| 2,00 | Propylene Glycol |
| 5,00 | Panthenol |
| 2,80 | Polymerisat gemäß Herstellbeispiel 7 |
| q.s. | Konservierungsmittel |
| 65,00 | dest. Wasser |

| Phase C | |
|---|---|
| q.s. | Parfümöl |
| 0,50 | Tocopheryl Acetate |

### Herstellung:

Phase A und B getrennt auf ca. 80°C erwärmen. Phase B in Phase A unter Homogenisieren einrühren, kurz nachhomogenisieren. Abkühlen auf ca. 40°C, Phase C hinzugeben, nochmals homogenisieren.

### Anwendungsbeispiel 31: Körperlotion-Schaum

| Phase A | |
|---|---|
| 1,50 | Ceteareth-25 |
| 1,50 | Ceteareth-6 |
| 4,00 | Cetearyl Alcohol |
| 10,00 | Cetearyl Octanoate |
| 1,00 | Dimethicone |

| Phase B | |
|---|---|
| 3,00 | Polymerisat gemäß Herstellbeispiel 2 |
| 2,00 | Panthenol |
| 2,50 | Propylene Glycol |
| q.s. | Konservierungsmittel |
| 74,50 | dest. Wasser |

| Phase C | |
|---|---|
| q.s. | Parfümöl |

### Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Abfüllung: 90 % Wirkstoff und 10 % Propan/Butan bei 3,5 bar (20°C).

### Anwendungsbeispiel 32: Gesichtswasser für trockene und empfindliche Haut

| Phase A | |
|---|---|
| 2,50 | PEG-40 Hydrogenated Castor Oil |
| q.s. | Parfümöl |
| 0,40 | Bisabolol |

| Phase B | |
|---|---|
| 3,00 | Glycerin |
| 1,00 | Hydroxyethyl Cetyldimonium Phosphate |
| 5,00 | Whitch Hazel (Hamamelis Virginiana) Distillate |
| 0,50 | Panthenol |
| 0,50 | Polymerisat gemäß Herstellbeispiel 25 |
| q.s. | Konservierungsmittel |
| 87,60 | dest. Wasser |

### Herstellung:

Phase A klar lösen. Phase B in Phase A einrühren.

### Anwendungsbeispiel 33: Gesichtswaschpaste mit Peelingeffekt

| Phase A | |
|---|---|
| 70,00 | dest. Wasser |
| 3,00 | Polymerisat gemäß Herstellbeispiel 15 |
| 1,50 | Carbomer |
| q.s. | Konservierungsmittel |

| Phase B | |
|---|---|
| q.s. | Parfümöl |
| 7,00 | Potassium Cocoyl Hydrolyzed Protein |
| 4,00 | Cocamidopropyl Betaine |

| Phase C | |
|---|---|
| 1,50 | Triethanolamine |

| Phase D | |
|---|---|
| 13,00 | Polyethylene (Luwax A™ von BASF) |

### Herstellung:

Phase A quellen lassen. Phase B klar lösen. Phase B in Phase A einrühren. Mit Phase C neutralisieren. Danach Phase D einrühren.

### Anwendungsbeispiel 34: Gesichtsseife

| Phase A | |
|---|---|
| 25.0 | Potassium Cocoate |
| 20.0 | Disodium Cocoamphodiacetate |
| 2.0 | Lauramide DEA |
| 1.0 | Glycol Stearate |
| 2.0 | Polymerisat gemäß Herstellbeispiel 23 |
| 50.0 | dest. Wasser |
| q.s. | Citric Acid |

| Phase B | |
|---|---|
| q.s. | Konservierungsmittel |
| q.s. | Parfumöl |

### Herstellung:

Phase A unter Rühren auf 70°C erwärmen, bis alles homogen ist. pH-Wert auf 7.0 bis 7.5 mit Citric Acid. Alles auf 50°C abkühlen lassen und Phase B zugeben.

### Anwendungsbeispiel 35: Gesichtsreinigungsmilch Typ O/W

| Phase A | |
|---|---|
| 1,50 | Ceteareth-6 |
| 1,50 | Ceteareth-25 |
| 2,00 | Glyceryl Stearate |
| 2,00 | Cetyl Alcohol |
| 10,00 | Mineral Oil |

| Phase B | |
|---|---|
| 5,00 | Propylene Glycol |
| q.s. | Konservierungsmittel |
| 1,0 | Polymerisat gemäß Herstellbeispiel 29 |
| 66,30 | dest. Wasser |

| Phase C | |
|---|---|
| 0,20 | Carbomer |
| 10,00 | Cetearyl Octanoate |

| Phase D | |
|---|---|
| 0,40 | Tetrahydroxypropyl Ethylenediamine |

| Phase E | |
|---|---|
| q.s. | Parfümöl |
| 0,10 | Bisabolol |

### Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

### Anwendungsbeispiel 36: Transparente Seife

| | |
|---|---|
| 4,20 | Sodium Hydroxide |
| 3,60 | dest. Wasser |
| 2,0 | Polymerisat gemäß Herstellbeispiel 32 |
| 22,60 | Propylene Glycol |
| 18,70 | Glycerin |
| 5,20 | Cocoamide DEA |
| 10,40 | Cocamine Oxide |
| 4,20 | Sodium Lauryl Sulfate |
| 7,30 | Myristic Acid |
| 16,60 | Stearic Acid |
| 5,20 | Tocopherol |

### Herstellung:

Alle Zutaten mischen. Die Mischung klar schmelzen bei 85°C. Sofort in die Form ausgiessen.

### Anwendungsbeispiel 37: Peeling-Creme, Typ O/W

| Phase A | |
|---|---|
| 3,00 | Ceteareth-6 |
| 1,50 | Ceteareth-25 |
| 3,00 | Glyceryl Stearate |
| 5,00 | Cetearyl Alcohol, Sodium Cetearyl Sulfate |
| 6,00 | Cetearyl Octanoate |
| 6,00 | Mineral Oil |
| 0,20 | Bisabolol |

| Phase B | |
|---|---|
| 2,00 | Propylene Glycol |
| 0,10 | Disodium EDTA |
| 3,00 | Polymerisat gemäß Herstellbeispiel 33e |
| q.s. | Konservierungsmittel |
| 59,70 | dest. Wasser |

| Phase C | |
|---|---|
| 0,50 | Tocopheryl Acetate |
| q.s. | Parfümöl |

| Phase D | |
|---|---|
| 10,00 | Polyethylene |

### Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Anschließend Phase D unterrühren.

### Anwendungsbeispiel 38: Rasierschaum

| | |
|---|---|
| 6,00 | Ceteareth-25 |
| 5,00 | Poloxamer 407 |
| 52,00 | dest. Wasser |
| 1,00 | Triethanolamine |
| 5,00 | Propylene Glycol |
| 1,00 | PEG-75 Lanolin Oil |
| 5,00 | Polymerisat gemäß Herstellbeispiel 5 |
| q.s. | Konservierungsmittel |
| q.s. | Parfümöl |
| 25,00 | Sodium Laureth Sulfate |

### Herstellung:

Alles zusammennwiegen, danach rühren bis gelöst. Abfüllung: 90 Teile Wirksubstanz und 10 Teile Propan/Butan-Mischung 25:75.

### Anwendungsbeispiel 39: After Shave Balsam

| Phase A | |
|---|---|
| 0,25 | Acrylates/C10-30 Alkyl Acrylate Crosspolymer |
| 1,50 | Tocopheryl Acetate |
| 0,20 | Bisabolol |
| 10,00 | Caprylic/Capric Triglyceride |
| q.s. | Parfümöl |
| 1,00 | PEG-40 Hydrogenated Castor Oil |

| Phase B | |
|---|---|
| 1,00 | Panthenol |
| 15,00 | Alcohol |
| 5,00 | Glycerin |
| 0,05 | Hydroxyethyl Cellulose |
| 1,92 | Polymerisat gemäß Herstellbeispiel 2 |
| 64,00 | dest. Wasser |

| Phase C | |
|---|---|
| 0,08 | Sodium Hydroxide |

### Herstellung:

Die Komponenten der Phase A mischen. Phase B unter Homogenisieren in Phase A einrühren, kurz nachhomogenisieren. Mit Phase C neutralisieren und erneut homogenisieren.

### Anwendungsbeispiel 40: Körperpflegecreme

| Phase A | |
|---|---|
| 2,00 | Ceteareth-6 |
| 2,00 | Ceteareth-25 |
| 2,00 | Cetearyl Alcohol |
| 3,00 | Glyceryl Stearate SE |
| 5,00 | Mineral Oil |
| 4,00 | Jojoba (Buxus Chinensis) Oil |
| 3,00 | Cetearyl Octanoate |
| 1,00 | Dimethicone |
| 3,00 | Mineral Oil, Lanolin Alcohol |

| Phase B | |
|---|---|
| 5,00 | Propylene Glycol |
| 0,50 | Veegum |
| 1,00 | Panthenol |
| 1,70 | Polymerisat gemäß Herstellbeispiel 4 |
| 6,00 | Polyquaternium-44 |
| q.s. | Konservierungsmittel |
| 60,80 | dest. Wasser |

| Phase C | |
|---|---|
| q.s. | Parfümöl |

### Herstellung:

Die Phasen A und B getrennt auf ca. 80°C erwärmen. Phase B homogenisieren.
Phase B unter Homogenisieren in Phase A einrühren, kurz nachhomogenisieren.
Abkühlen auf ca. 40°C, Phase C hinzugeben und nochmals kurz homogenisieren.

### Anwendungsbeispiel 41: Zahnpasta

| Phase A | |
|---|---|
| 34,79 | dest. Wasser |
| 3,00 | Polymerisat gemäß Herstellbeispiel 31 |
| 0,30 | Konservierungsmittel |
| 20,00 | Glycerin |
| 0,76 | Sodium Monofluorophosphate |

| Phase B | |
|---|---|
| 1,20 | Sodium Carboxymethylcellulose |

| Phase C | |
|---|---|
| 0,80 | Aromaöl |
| 0,06 | Saccharin |
| 0,10 | Konservierungsmittel |
| 0,05 | Bisabolol |
| 1,00 | Panthenol |
| 0,50 | Tocopheryl Acetate |
| 2,80 | Silica |
| 1,00 | Sodium Lauryl Sulfate |
| 7,90 | Dicalciumphosphate Anhydrate |
| 25,29 | Dicalciumphosphate Dihydrate |
| 0,45 | Titanium Dioxide |

### Herstellung:

Phase A lösen. Phase B in Phase A einstreuen und lösen. Phase C zugeben und unter Vakuum bei RT ca. 45 Min. rühren lassen.

### Anwendungsbeispiel 42: Mundwasser

| Phase A | |
|---|---|
| 2,00 | Aromaöl |
| 4,00 | PEG-40 Hydrogenated Castor Oil |
| 1,00 | Bisabolol |
| 30,00 | Alcohol |

| Phase B | |
|---|---|
| 0,20 | Saccharin |
| 5,00 | Glycerin |
| q.s. | Konservierungsmittel |
| 5,00 | Poloxamer 407 |
| 0,5 | Polymerisat gemäß Herstellbeispiel 7 |
| 52,30 | dest. Wasser |

### Herstellung:

Phase A und Phase B getrennt klar lösen. Phase B in Phase A einrühren.

### Anwendungsbeispiel 43: Prothesenhaftmittel

| Phase A | |
|---|---|
| 0,20 | Bisabolol |
| 1,00 | Beta-Carotene |
| q.s. | Aromaöl |
| 20,00 | Cetearyl Octanoate |
| 5,00 | Silica |
| 33,80 | Mineral Oil |

| Phase B | |
|---|---|
| 5,00 | Polymerisat gemäß Herstellbeispiel 15 |
| 35,00 | PVP (20%ige Lösung in Wasser) |

### Herstellung:

Phase A gut mischen. Phase B in Phase A einrühren Anwendungsbeispiel 32: Hautpflegecreme, Typ O/W

| Phase A | |
|---|---|
| 8,00 | Cetearyl Alcohol |
| 2,00 | Ceteareth-6 |
| 2,00 | Ceteareth-25 |
| 10,00 | Mineral Oil |
| 5,00 | Cetearyl Octanoate |
| 5,00 | Dimethicone |

| Phase B | |
|---|---|
| 3,00 | Polymerisat gemäß Herstellbeispiel 19 |
| 2,00 | Panthenol, Propylene Glycol |
| q.s. | Konservierungsmittel |
| 63,00 | dest. Wasser |

| Phase C | |
|---|---|
| q.s. | Parfümöl |

### Herstellung:

Phase A und B getrennt auf ca. 80 C erwärmen. Phase B in Phase A unter Homogenisieren einrühren, kurz nachhomogenisieren. Abkühlen auf ca. 40 C, Phase C hinzugeben, nochmals homogenisieren.

### Anwendungsbeispiel 44: Hautpflegecreme, Typ W/O

| Phase A | |
|---|---|
| 6,00 | PEG-7 Hydrogenated Castor Oil |
| 8,00 | Cetearyl Octanoate |
| 5,00 | Isopropyl Myristate |
| 15,00 | Mineral Oil |
| 2,00 | PEG-45/Dodecyl Glycol Copolymer |
| 0,50 | Magnesium Stearate |
| 0,50 | Aluminum Stearate |

| Phase B | |
|---|---|
| 3,00 | Glycerin |
| 3,30 | Polymerisat gemäß Herstellbeispiel 10 |
| 0,70 | Magnesium Sulfate |
| 2,00 | Panthenol |
| q.s. | Konservierungsmittel |
| 48,00 | dest. Wasser |

| Phase C | |
|---|---|
| 1,00 | Tocopherol |
| 5,00 | Tocopheryl Acetate |
| q.s. | Parfümöl |

### Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren.

### Anwendungsbeispiel 45: Lippenpflegecreme

| Phase A | |
|---|---|
| 10,00 | Ceteäryl Octanoate |
| 5,00 | Polybutene |

| Phase B | |
|---|---|
| 0,10 | Carbomer |

| Phase C | |
|---|---|
| 2,00 | Ceteareth-6 |
| 2,00 | Ceteareth-25 |
| 2,00 | Glyceryl Stearate |
| 2,00 | Cetyl Alcohol |
| 1,00 | Dimethicone |
| 1,00 | Benzophenone-3 |
| 0,20 | Bisabolol |
| 6,00 | Mineral Oil |

| Phase D | |
|---|---|
| 8,00 | Polymerisat gemäß Herstellbeispiel 33a |
| 3,00 | Panthenol |
| 3,00 | Propylene Glycol |
| q.s. | Konservierungsmittel |
| 54,00 | dest. Wasser |

| Phase E | |
|---|---|
| 0,10 | Triethanolamine |

| Phase F | |
|---|---|
| 0,50 | Tocopheryl Acetate |
| 0,10 | Tocopherol |
| q.s. | Parfümöl |

### Herstellung:

Phase A klar lösen. Phase B dazugeben und homogenisieren. Phase C addieren und schmelzen bei 80 C. Phase D auf 80 C erwärmen. Phase D zu Phase ABC geben und homogenisieren. Abkühlen auf ca. 40 C, Phase E und Phase F zugeben, nochmals homogenisieren.

### Anwendungsbeispiel 46: Glänzender Lippenstift

| Phase A | |
|---|---|
| 5,30 | Candelilla (Euphorbia Cerifera) Wax |
| 1,10 | Bees Wax |
| 1,10 | Microcrystalline Wax |
| 2,00 | Cetyl Palmitate |
| 3,30 | Mineral Oil |
| 2,40 | Castor Oil, Glyceryl Ricinoleate, Octyldodecanol, Carnauba, Candelilla Wax, |
| 0,40 | Bisabolol |
| 16,00 | Cetearyl Octanoate |
| 2,00 | Hydrogenated Coco-Glycerides |
| q.s. | Konservierungsmittel |
| 1,00 | Polymerisat gemäß Herstellbeispiel 33e |
| 60,10 | Castor (Ricinus Communis) Oil |
| 0,50 | Tocopheryl Acetate |

| Phase B | |
|---|---|
| 0,80 | C. I. 14 720:1, Acid Red 14 Aluminum Lake |

| Phase C | |
|---|---|
| 4,00 | Mica, Titanium Dioxide |

### Herstellung:

Die Komponenten der Phase A einwiegen und aufschmelzen. Phase B homogen einarbeiten. Phase C zugeben und unterrühren. Unter Rühren auf Raumtemperatur abkühlen.

## Patentansprüche

1. Verwendung von Pfropfcopolymerisaten, die erhältlich sind durch radikalische Pfropfcopolymerisation von
a) mindestens einer offenkettigen N-Vinylamidverbindung der allgemeinen Formel (I) 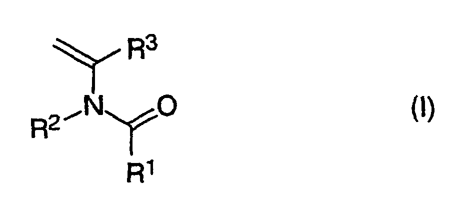 wobei R¹, R², R³ = H oder C₁- bis C₆-Alkyl bedeuten, und
b) gegebenenfalls eines oder mehreren weiteren copolymerisierbaren Monomeren
auf eine polymere Pfropfgrundlage c), für kosmetische Anwendungen, mit der Maßgabe, daß falls die polymere Pfropfgrundlage eine polyetherhaltige Verbindung ist, das copolymerisierbare Polymer b) kein Vinylester darstellt.

2. Verwendung nach Anspruch 1, **dadurch gekennzeichnet, daß** die Pfropfpolymerisate wasserlöslich oder wasserdispergierbar sind.

3. Verwendung nach Anspruch 1, wobei die Reste R¹, R² und R³ in Formel (I) = H bedeuten.

4. Verwendung von wasserlöslichen oder wasserdispergierbaren Pfropfcopolymerisaten nach Anspruch 1, **dadurch gekennzeichnet, daß** als c) ausgewählt ist aus
c1) polyetherhaltigen Verbindungen
c2) Polymerisaten, die mindestens 5 Gew.% an Vinylpyrrolidon-Einheiten enthalten
c3) Polymerisaten, die mindestens 50 Gew.% an Vinylalkohol-Einheiten enthalten
und/oder
c4) natürlichen Substanzen, die Saccharid-Strukturen enthalten.

5. Verwendung von wasserlöslichen oder wasserdispergierbaren Pfropfcopolymerisaten nach Anspruch 4, **dadurch gekennzeichnet, daß** die polyetherhaltige Verbindung c1) ausgewählt aus Polymerisaten der allgemeinen Formel II mit einem mittleren Molekulargewicht von > 300 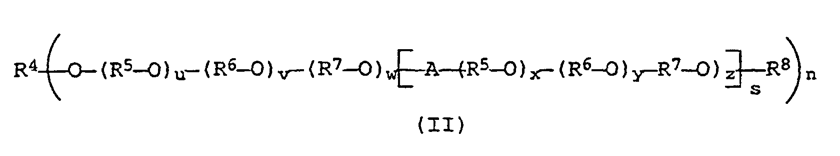 in der die Variablen unabhängig voneinander folgende Bedeutung haben:
R⁴ Wasserstoff, C₁-C₂₄-Alkyl, R⁹-C(=O)-, R⁹-NH-C(=O)-, Polyalkoholrest;
R⁸ Wasserstoff, C₁-C₂₄-Alkyl, R⁹-C(=O)-, R⁹-NH-C(=O)-;
R⁵ bis R⁷ -(CH₂)₂-, -(CH₂)₃-, -(CH₂)₄-, -CH₂-CH(R⁹)-, -CH₂-CHOR¹⁴-CH₂-;
R⁹ C₁-C₂₄-Alkyl;
R¹⁰ Wasserstoff, C₁-C₂₄-Alkyl, R⁹-C(=O)-, R⁹-NH-C(=O)-;
A -C(=O)-, -C(=O)-B-C(=O)-O, -C(=O)-NH-B-NH-C(=O)-O;
B -(CH₂)ₜ-, Arylen, ggf. substituiert;
n 1 bis 1000;
s 0 bis 1000;
t 1 bis 12;
u 1 bis 5000;
v 0 bis 5000;
w 0 bis 5000;
x 0 bis 5000;
y 0 bis 5000;
z 0 bis 5000.

6. Verwendung von wasserlöslichen oder wasserdispergierbaren Pfropfcopolymerisaten nach Anspruch 5, **dadurch gekennzeichnet, daß** die polyetherhaltige Verbindung c1) ausgewählt ist aus Polymerisaten der allgemeinen Formel II mit einem mittleren Molekulargewicht von 300 bis 100000 (nach dem Zahlenmittel), in der die Variablen unabhängig voneinander folgende Bedeutung haben:
R⁴ Wasserstoff, C₁-C₁₂-Alkyl, R⁹-C(=O)-, R⁹-NH-C(=O)-, Polyalkoholrest;
R⁸ Wasserstoff, C₁-C₁₂-Alkyl, R⁹-C(=O)-, R⁹-NH-C(=O)-;
R⁵ bis R⁷ -(CH₂)₂-, -(CH₂)₃-, -(CH₂)₄-, -CH₂-CH(R⁹)-, -CH₂-CHOR¹⁰-CH₂-;
R⁹ C₁-C₁₂-Alkyl;
R¹⁰ Wasserstoff, C₁-C₁₂-Alkyl. R⁹-C(=O)-, R⁹-NH-C(=O)-;
n 1 bis 8;
s 0;
u 2 bis 2000;
v 0 bis 2000;
w 0 bis 2000.

7. Verwendung von wasserlöslichen oder wasserdispergierbaren Pfropfcopolymerisaten nach Anspruch 4, **dadurch gekennzeichnet, daß** die polyetherhaltige Verbindung c1) ausgewählt aus Polymerisaten der allgemeinen Formel II mit einem mittleren Molekulargewicht von 500 bis 50000 (nach dem Zahlenmittel), in der die Variablen unabhängig voneinander folgende Bedeutung haben:
R⁴ Wasserstoff, C₁-C₆-Alkyl, R⁹-C(=O)-, R⁹-NH-C(=O)-;
R⁸ Wasserstoff, C₁-C₆-Alkyl , R⁹-C(=O)-, R⁹-NH-C(=O)-;
R⁵ bis R⁷ -(CH₂)₂-, -(CH₂)₃-, -(CH₂)₄-, -CH₂-CH(R⁹)-, -CH₂-CHOR¹⁰-CH₂-;
R⁹ C₁-C₆-Alkyl;
R¹⁰ Wasserstoff, C₁-C₆-Alkyl, R⁹-C(=O)-, R⁹-NH-C(=O)-;
n 1;
s 0;
u 5 bis 500;
v 0 bis 500;
w 0 bis 500.

8. Verwendung von wasserlöslichen oder wasserdispergierbaren Pfropfcopolymerisaten nach Anspruch 4, **dadurch gekennzeichnet, daß** die polyetherhaltige Verbindung c1) ausgewählt ist aus polyetherhaltigen Silikonderivaten

9. Verwendung von wasserlöslichen oder wasserdispergierbaren Pfropfcopolymerisaten nach Anspruch 4, **dadurch gekennzeichnet, daß** die polyetherhaltige Verbindung c1) ausgewählt ist aus polyetherhaltigen Silikonderivaten der allgemeinen Formel III 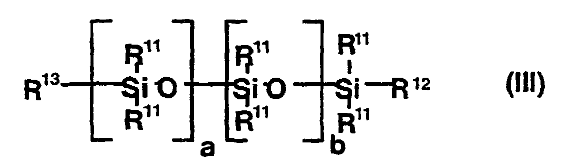 wobei:
R¹² = CH₃ oder 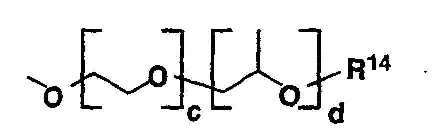
R¹³ = CH₃ oder R¹²
R¹⁴ = H, CH₃, 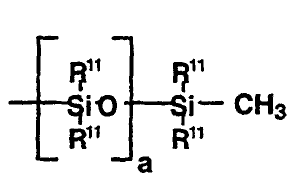 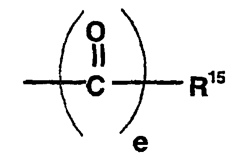
R¹⁵ ein organischer Rest aus 1 bis 40 Kohlenstoffatomen, der Amino-, Carbonsäure- oder Sulfonatgruppen enthalten kann, oder für den Fall e=0, auch das Anion einer anorganischen Säure bedeutet,
und wobei die Reste R¹¹ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich R¹⁶ sind, wobei: 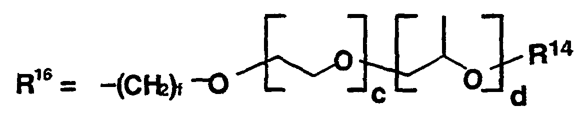 mit der Maßgabe, daß mindestens einer der Reste R¹¹, R¹² oder R¹³ ein polyalkylenoxidhaltiger Rest nach obengenannter Definition ist,
und f eine ganze Zahl von 1 bis 6 ist,
a und b ganze Zahlen derart sind, daß das Molekulargewicht des Polysiloxan-Blocks zwischen 300 und 30000 liegt,
c und d ganze Zahlen zwischen 0 und 50 sein können mit der Maßgabe, daß die Summe aus c und d größer als 0 ist, und e 0 oder 1 ist.

10. Verwendung von Polymerisaten nach Anspruch 9, **dadurch gekennzeichnet, daß** Formel III folgende Bedeutung besitzt:

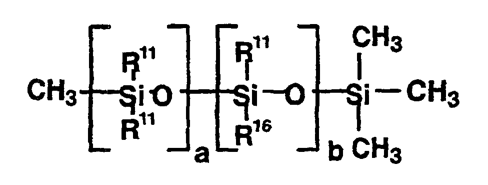

11. Verwendung von wasserlöslichen oder wasserdispergierbaren Pfropfcopolymerisaten nach Anspruch 4, **dadurch gekennzeichnet, daß** die polyetherhaltige Verbindung c1) ausgewählt ist aus Polymerisaten, die erhältlich durch Umsetzung von Polyethyleniminen mit Alkylenoxiden.

12. Verwendung von Polymerisaten nach Anspruch 4, **dadurch gekennzeichnet, daß** die polyetherhaltigen Verbindungen c1) durch Polymerisation von ethylenisch ungesättigten alkylenoxidhaltigen Monomeren und gegebenenfalls weiteren copolymerisierbaren Monomeren hergestellt worden sind.

13. Verwendung von Polymerisaten nach Anspruch 12, **dadurch gekennzeichnet, daß** die polyetherhaltigen Verbindungen c1) durch Polymerisation von Polyalkylenoxidvinylethern und gegebenenfalls weiteren copolymerisierbaren Monomeren hergestellt worden sind.

14. Verwendung von Polymerisaten nach Anspruch 12, **dadurch gekennzeichnet, daß** die polyetherhaltigen Verbindungen c1) durch Polymerisation von Polyalkylenoxid(meth)acrylaten und gegebenenfalls weiteren copolymerisierbaren Monomeren hergestellt worden sind.

15. Verwendung von wasserlöslichen oder wasserdispergierbaren Pfropfcopolymerisaten nach Anspruch 4, **dadurch gekennzeichnet, daß** als weitere Comonomere des N-Vinylpyrrolidons zur Synthese der Pfropfgrundlage c2) ausgewählt werden aus der Gruppe:
(N-Vinylcaprolactam, N-Vinylimidazol und alkylsubstituierte N-Vinylimidazole sowie deren Salze mit Carbonsäuren oder Mineralsäuren sowie deren quarternierter Produkte, ungesättigte Sulfonsäuren, Diallylammoniumchlorid, Vinylester, Vinylether, Styrol, Alkylstyrole, monoethylenisch ungesättigte Carbonsäure bzw. deren Salze, Ester, Amide und Nitrile, Maleinsäureanhydrid sowie dessen Hälbester, N,N-Dialkylaminoalkyl(meth)-acrylate, sowie deren Salze mit Carbonsäuren oder Mineralsäuren sowie die quarternierten Produkte.

16. Verwendung von wasserlöslichen oder wasserdispergierbaren Pfropfcopolymerisaten nach Anspruch 4, **dadurch gekennzeichnet, daß** die Pfropfgrundlage c4)ausgewählt ist aus Monosacchariden, Oligosacchariden, Polysacchariden, oxidativ, hydrolytisch oder enzymatisch abgebauten Polysacchariden, chemisch modifizierten Oligo- oder Polysacchariden und Gemischen davon.

17. Verwendung von Polymerisaten nach Ansprüchen 1, **dadurch gekennzeichnet, daß** als weitere Comonomere b) ausgewählt werden aus der Gruppe:
monoethylenisch ungesättigte Carbonsäuren und deren Salze, Ester, Amide und Nitrile von monoethylenisch ungesättigten Carbonsäuren, Maleinsäureanhydrid sowie dessen Halbester, Diallylammoniumchlorid, Vinylester, Styrol, Alkylstyrole, ungesättigte Sulfonsäuren, N-Vinyllactame, Vinylether, 1-Vinylimidazol und Alkylsubstituierte Vinylimidazole sowie deren Salze mit Carbonsäuren oder Mineralsäuren sowie deren quarternierten Produkte, N,N-Dialkylaminoalkyl(meth)acrylate sowie deren quarternierten Produkte.

18. Verwendung von Polymerisaten nach Anspruch 1, **dadurch gekennzeichnet, daß** die Polymerisate zumindest teilweise verseift werden.

## Claims

1. The use of graft copolymers obtainable by free-radical graft copolymerization of
a) at least one open-chain N-vinylamide compound of the formula (I) 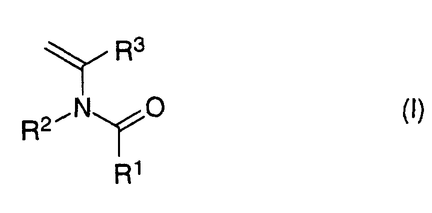 where R¹, R², R³ = H or C₁-C₆-alkyl,
and
b) optionally one or more further copolymerizable monomers
to a polymeric graft base c), for cosmetic applications, with the proviso that if the polymeric graft base is a polyether-containing compound, the copolymerizable polymer b) is not a vinyl ester.

2. The use as claimed in claim 1, wherein the graft polymers are water-soluble or water-dispersible.

3. The use as claimed in claim 1, where the radicals R¹, R² and R³ in formula (I) = H.

4. The use of water-soluble or water-dispersible graft copolymers as claimed in claim 1, wherein c) is chosen from
c1) polyether-containing compounds
c2) polymers which contain at least 5% by weight of vinylpyrrolidone units
c3) polymers which contain at least 50% by weight of vinyl alcohol units
and/or
c4) natural substances which contain saccharide structures.

5. The use of water-soluble or water-dispersible graft copolymers as claimed in claim 4, wherein the polyether-containing compound c1) is chosen from polymers of the formula II having an average molecular weight of > 300 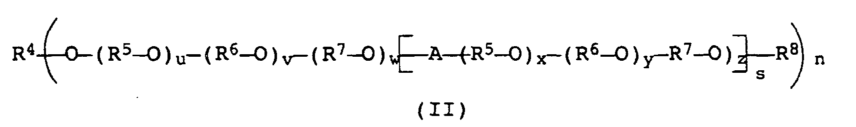 in which the variables, independently of one another, have the following meanings:
R⁴ is hydrogen, C₁-C₂₄-alkyl, R⁹-C(=O)-, R⁹-NH-C(=O)-, polyalcohol radical;
R⁸ is hydrogen, C₁-C₂₄-alkyl, R⁹-C(=O)-, R⁹-NH-C(=O)-;
R⁵ to R⁷ are -(CH₂)₂-, -(CH₂)₃-, -(CH₂)₄-, -CH₂-CH(R⁹)-, -CH₂-CHOR¹⁰-CH₂-;
R⁹ is C₁-C₂₄-alkyl;
R¹⁰ is hydrogen, C₁-C₂₄-alkyl, R⁹-C(=O)-, R⁹-NH-C(=O)-;
A is -C(=O)-O, -C(=O)-B-C(=O)-O, -C(=O)-NH-B-NH-C(=O)-O;
B is -(CH₂)ₜ-, arylene, optionally substituted;
n is 1 to 1000;
s is 0 to 1000;
t is 1 to 12;
u is 1 to 5000;
v is 0 to 5000;
w is 0 to 5000;
x is 0 to 5000;
y is 0 to 5000;
z is 0 to 5000.

6. The use of water-soluble or water-dispersible graft copolymers as claimed in claim 5, wherein the polyether-containing compound c1) is chosen from polymers of the formula II having an average molecular weight of from 300 to 100,000 (according to the number average), in which the variables, independently of one another, have the following meanings:
R⁴ is hydrogen, C₁-C₁₂-alkyl, R⁹-C(=O)-, R⁹-NH-C(=O)-, polyalcohol radical;
R⁸ is hydrogen, C₁-C₁₂-alkyl, R⁹-C(=O)-, R⁹-NH-C(=O)-;
R⁵ to R⁷ are -(CH₂)₂-, -(CH₂)₃-, -(CH₂)₄-, -CH₂-CH(R⁹)-, -CH₂-CHOR¹⁰-CH₂-;
R⁹ is C₁-C₁₂-alkyl;
R¹⁰ is hydrogen, C₁-C₁₂-alkyl, R⁹-C(=O)-, R⁹-NH-C(=O)-;
n is 1 to 8;
s is 0;
u is 2 to 2000;
v is 0 to 2000;
w is 0 to 2000.

7. The use of water-soluble or water-dispersible graft copolymers as claimed in claim 4, wherein the polyether-containing compound c1) is chosen from polymers of the formula II having an average molecular weight of from 500 to 50,000 (according to the number average), in which the variables, independently of one another, have the following meanings:
R⁴ is hydrogen, C₁-C₆-alkyl, R⁹-C(=O)-, R⁹-NH-C(=O)-;
R⁸ is hydrogen, C₁-C₆-alkyl, R⁹-C(=O)-, R⁹-NH-C(=O)-;
R⁵ to R⁷ are -(CH₂)₂-, -(CH₂)₃-, -(CH₂)₄-, -CH₂-CH(R⁹)-, -CH₂-CHOR¹⁰-CH₂-;
R⁹ is C₁-C₆-alkyl;
R¹⁰ is hydrogen, C₁-C₆-alkyl, R⁹-C(=O)-, R⁹-NH-C(=O)-;
n is 1;
s is 0;
u is 5 to 500;
v is 0 to 500;
w is 0 to 500.

8. The use of water-soluble or water-dispersible graft copolymers as claimed in claim 4, wherein the polyether-containing compound c1) is chosen from polyether-containing silicone derivatives.

9. The use of water-soluble or water-dispersible graft copolymers as claimed in claim 4, wherein the polyether-containing compound c1) is chosen from polyether-containing silicone derivatives of the formula III 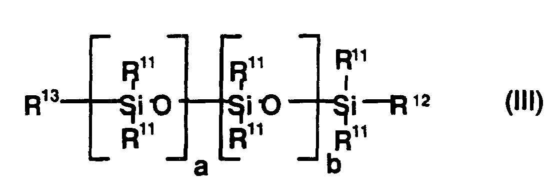 where:
R¹² = CH₃ or 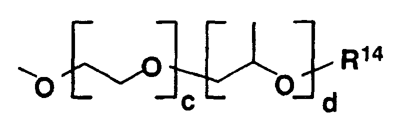
R¹³ = CH₃ or R¹²
R¹⁴ **=** H, CH₃, 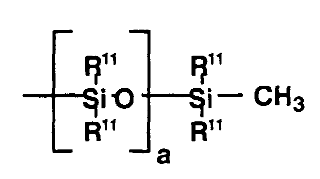 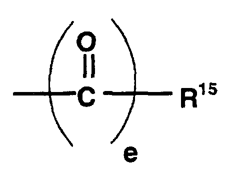
R¹⁵ is a C₁-C₄₀ organic radical which can contain amino, carboxylic acid or sulfonate groups, or for the case e=0, is also the anion of an inorganic acid,
and where the radicals R¹¹ may be identical or different, and either originate from the group of aliphatic hydrocarbons having 1 to 20 carbon atoms, are cyclic aliphatic hydrocarbons having 3 to 20 carbon atoms, are of an aromatic nature or are identical to R¹⁶, where: 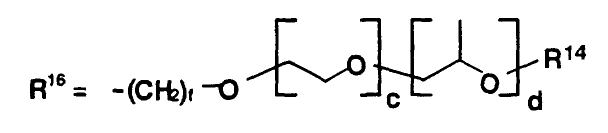 with the proviso that at least one of the radicals R¹¹, R¹² or R¹³ is a polyalkylene-oxide-containing radical according to the abovementioned definition,
and f is an integer from 1 to 6,
a and b are integers such that the molecular weight of the polysiloxane block is between 300 and 30 000,
c and d may be integers between 0 and 50, with the proviso that the sum of c and d is greater than 0, and e is 0 or 1.

10. The use of polymers as claimed in claim 9, wherein formula III has the following meaning:

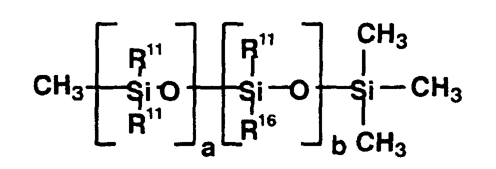

11. The use of water-soluble or water-dispersible graft copolymers as claimed in claim 4, wherein the polyether-containing compound c1) is chosen from polymers obtainable by reacting polyethyleneimines with alkylene oxides.

12. The use of polymers as claimed in claim 4, wherein the polyether-containing compounds c1) have been prepared by polymerization of ethylenically unsaturated alkylene-oxide-containing monomers and optionally further copolymerizable monomers.

13. The use of polymers as claimed in claim 12, wherein the polyether-containing compounds c1) have been prepared by polymerization of polyalkylene oxide vinyl ethers and optionally further copolymerizable monomers.

14. The use of polymers as claimed in claim 12, wherein the polyether-containing compounds c1) have been prepared by polymerization of polyalkylene oxide (meth)acrylates and optionally further copolymerizable monomers.

15. The use of water-soluble or water-dispersible graft copolymers as claimed in claim 4, wherein further comonomers of N-vinylpyrrolidone for the synthesis of the graft base c2) are chosen from the group:
N-vinylcaprolactam, N-vinylimidazole and alkyl-substituted N-vinylimidazoles and salts thereof with carboxylic acids or mineral acids, and quaternized products thereof, unsaturated sulfonic acids, diallylammonium chloride, vinyl esters, vinyl ethers, styrene, alkylstyrenes, monoethylenically unsaturated carboxylic acid and salts, esters, amides and nitriles thereof, maleic anhydride and its monoester, N,N-dialkylaminoalkyl (meth)acrylates, and salts thereof with carboxylic acids or mineral acids, and the quaternized products.

16. The use of water-soluble or water-dispersible graft copolymers as claimed in claim 4, wherein the graft base c4) is chosen from monosaccharides, oligosaccharides, polysaccharides, oxidatively, hydrolytically or enzymatically degraded polysaccharides, chemically modified oligosaccharides or polysaccharides and mixtures thereof.

17. The use of polymers as claimed in claim 1, wherein the further comonomers b) are chosen from the group:
monoethylenically unsaturated carboxylic acids and salts thereof, esters, amides and nitriles of monoethylenically unsaturated carboxylic acids, maleic anhydride and its monoester, diallylammonium chloride, vinyl esters, styrene, alkylstyrenes, unsaturated sulfonic acids, N-vinyllactams, vinyl ethers, 1-vinylimidazole and alkyl-substituted vinylimidazoles and salts thereof with carboxylic acids or mineral acids, and quaternized products thereof, N,N-dialkylaminoalkyl (meth)acrylates and quaternized products thereof.

18. The use of polymers as claimed in claim 1, wherein the polymers are at least partially saponified.

## Revendications

1. Utilisation de copolymères greffés, que l'on peut obtenir par copolymérisation de greffage radicalaire de
a) au moins un composé de N-vinylamide à chaîne ouverte de la formule générale (I) 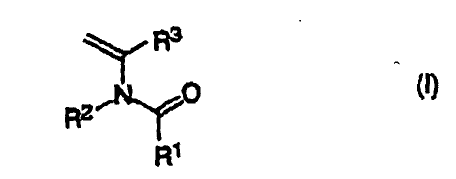 dans laquelle R¹, R², R³ = H ou représentent des radicaux alkyle en C₁ à C₆,
et
b) éventuellement un ou plusieurs autres monomères copolymérisables
sur une base de greffage polymère c), pour des applications cosmétiques, à la condition que lorsque la base de greffage polymère est un composé contenant un polyéther, le polymère copolymérisable b) ne soit pas un ester vinylique.

2. Utilisation selon la revendication 1, **caractérisée en ce que** les polymères greffés sont solubles dans l'eau ou dispersibles dans l'eau.

3. Utilisation selon la revendication 1, où les restes R¹, R² et R³ dans la formule (I) représentent H.

4. Utilisation de polymères greffés solubles dans l'eau ou dispersibles dans l'eau selon la revendication 1, **caractérisée en ce que** l'on choisit en tant que c) parmi
c1) des composés contenant un polyéther
c2) des polymères contenant au moins 5% en poids d'unités de vinylpyrrolidone
c3) des polymères contenant au moins 50% en poids d'unités d'alcool vinylique
et/ou
c4) des substances naturelles contenant des structures de saccharide.

5. Utilisation de polymères greffés solubles dans l'eau ou dispersibles dans l'eau selon la revendication 4, **caractérisée en ce que** le composé c1) contenant un polyéther est choisi parmi des polymères de la formule générale II d'un poids moléculaire moyen supérieur à 300 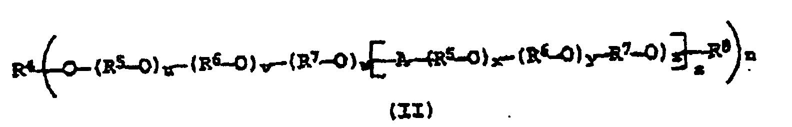 où les variables ont indépendamment les unes des autres la signification suivante:
R⁴ hydrogène, alkyle en C₁ à C₂₄, R⁹-C(=O)-, R⁹-NH-C(=O)-, reste de polyalcool,
R⁸ hydrogène, alkyle en C₁ à C₂₄, R⁹-C(=O)-, R⁹-NH-C(=O)-,
R⁵ à R⁷ -(CH₂)₂-, -(CH₂)₃-, -(CH₂)₄-. -CH₂-CH(R⁹)-, -CH₂-CHOR¹⁰-CH₂- ,
R⁹ alkyle en C₁ à C₂₄,
R¹⁰ hydrogène, alkyle en C₁ à C₂₄, R⁹-C(=O)-, R⁹-NH-C(=O)-,
A -C(=O)-O, -C(=O)-B-C(=O)-O, -C(=O)-NH-B-NH-C(=O)-O,
B -(CH₂)ₜ-, arylène, éventuellement substitué,
n 1 à 1000,
s 0 à 1000,
t 1 à 12,
u 1 à 5000,
v 0 à 5000,
w 0 à 5000,
x 0 à 5000,
y 0 à 5000,
z 0 à 5000.

6. Utilisation de polymères greffés solubles dans l'eau ou dispersibles dans l'eau selon la revendication 5, **caractérisée en ce que** le composé c1) contenant un polyéther est choisi parmi des polymères de la formule générale II d'un poids moléculaire moyen de 300 à 100.000 (moyenne numérique), où les variables ont indépendamment les unes des autres la signification suivante:
R⁴ hydrogène, alkyle en C₁ à C₁₂, R⁹-C (=O)-, R⁹-NH-C(=O)-, reste de polyalcool,
R⁸ hydrogène, alkyle en C₁ à C₁₂, R⁹-C(=O)-, R⁹-NH-C(=O)-,
R⁵ à R⁷ -(CH₂)₂-, -(CH₂)₃-, -(CH₂)₄-, -CH₂-CH(R⁹)-, -CF₂-CHOR¹⁰-CH₂-,
R⁹ alkyle en C₁ à C₁₂,
R¹⁰ hydrogène, alkyle en C₁ à C₂₂, R⁹-C(=)-, R⁹-NH-C(=O)-,
n 1 à 8,
s 0,
u 2 à 2000,
v 0 à 2000,
w 0 à 2000.

7. Utilisation de polymères greffés solubles dans l'eau ou dispersibles dans l'eau selon la revendication 4, **caractérisée en ce que** le composé c1) contenant un polyéther est choisi parmi des polymères de la formule générale II d'un poids moléculaire moyen de 500 à 50.000 (moyenne numérique), où les variables ont indépendamment les unes des autres la signification suivante:
R⁴ hydrogène, alkyle en C₁ à C₆, R⁹-C(=O)-, R⁹-NH-C(=O)-,
R⁸ hydrogène, alkyle en C₁ à C₆, R⁹-C(=O)-, R⁹-NH-C(=O)-,
R⁵ à R⁷ -(CH₂)₂-, -(CH₂)₃-, -(CH₂)₄-, -CH₂-CH(R⁹)-, -CH₂-CHOR¹⁰-CH₂-,
R⁹ alkyle en C₁ à C₆,
R¹⁰ hydrogène, alkyle en C₁ à C₆, R⁹-C(=O)-, R⁹-NH-C(=O)-,
n 1,
s 0,
u 5 à 500,
v 0 à 500,
w 0 à 500.

8. Utilisation de polymères greffés solubles dans l'eau ou dispersibles dans l'eau selon la revendication 4, **caractérisée en ce que** le composé c1) contenant un polyéther est choisi parmi des dérivés siliconés contenant un polyéther.

9. Utilisation de polymères greffés solubles dans l'eau ou dispersibles dans l'eau selon la revendication 4, **caractérisée en ce que** le composé c1) contenant un polyéther est choisi parmi des dérivés siliconés contenant un polyéther, de la formule générale III 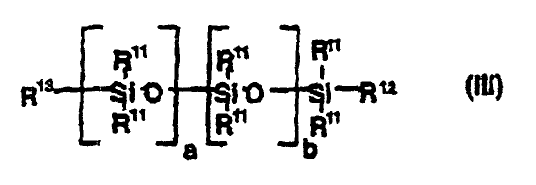 dans laquelle
R¹² = CH₃ ou 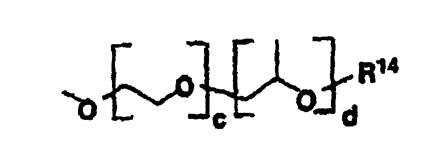
R¹³ = CH₃ ou R¹² ,
R¹⁴ + H, CH₃, 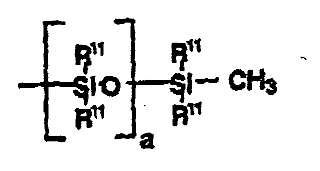 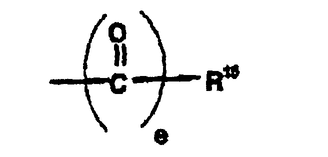
R¹⁵ représente un reste organique constitué de 1 à 40 atomes de carbone, qui peut contenir des groupes amino, acide carboxylique ou sulfonate, ou bien, dans le cas où e = 0, représente aussi l'anion d'un acide inorganique,
et où les restes R¹¹ peuvent être identiques ou différents et soit proviennent, du groupe des hydrocarbures aliphatiques comportant de 1 à 20 atomes de carbone, sont des hydrocarbures aliphatiques cycliques comportant de 3 à 20 atomes de carbone, sont de nature aromatique, ou sont identiques à R¹⁶, où 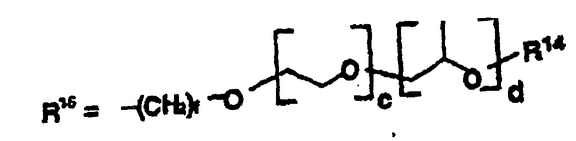 à la condition qu'au moins l'un des restes R¹¹, R¹² ou R¹³ soit un reste contenant du poly(oxyde d'alkylène) selon la définition ci-avant,
et que f soit un nombre entier de 1 à 6,
a et b étant des nombres entiers tels que le poids moléculaire du bloc de polysiloxane soit entre 300 et 30000,
c et d peuvent être des nombres entiers entre 0 et 50, à la condition que la somme de c et d soit supérieure à 0, et que e ait une valeur de 0 ou 1.

10. Utilisation de polymères selon la revendication 9, **caractérisée en ce que** la formule III a la signification suivante:

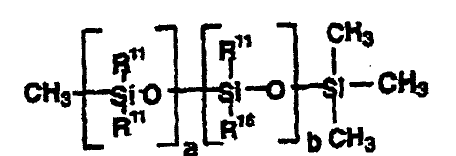

11. Utilisation de polymères greffés solubles dans l'eau ou dispersibles dans l'eau selon la revendication 4, **caractérisée en ce que** le composé c1) contenant un polyéther est choisi parmi des polymères que l'on peut obtenir par réaction de polyéthylène imines avec des oxydes d'alkylène.

12. utilisation de polymères selon la revendication 4, **caractérisée en ce que** les composés c1) contenant un polyéther ont été préparés par polymérisation de monomères éthyléniquement insaturés contenant de l'oxyde d'alkylène et éventuellement d'autres monomères copolymérisables.

13. Utilisation de polymères selon la revendication 12, **caractérisée en ce que** les composés c1) contenant un polyéther ont été préparés par polymérisation d'éthers vinyliques de poly(oxydes d'alkylène) et éventuellement d'autres monomères copolymérisables.

14. Utilisation de polymères selon la revendication 12, **caractérisée en ce que** les composés c1) contenant un polyéther ont été préparés par polymérisation de (méth)acrylates de poly(oxydes d'alkylène) et éventuellement d'autres monomères copolymérisables.

15. Utilisation de polymères greffés solubles dans l'eau ou dispersibles dans l'eau selon la revendication 4, **caractérisée en ce que** l'on choisit comme autres comonomères de la N-vinylpyrrolidone pour la synthèse de la base de greffage c2) dans le groupe formé par: le N-vinylcaprolactame, le N-vinylimidazole et des N-vinylimidazoles alkylsubstitués, ainsi que leurs sels avec des acides carboxyliques ou des acides minéraux, ainsi que leurs produits quaternisés, des acides sulfoniques insaturés, du chlorure de diallylammonium, des esters vinyliques, des éthers vinyliques, du styrène, des alkylstyrènes, des acides carboxyliques monoéthyléniquement insaturés ou leurs sels, esters, amides et nitriles, l'anhydride maléique et ses semi-esters, des (méth)acrylates de N,N-dialkylaminoalkyle, ainsi que leurs sels avec des acides carboxyliques ou des acides minéraux, ainsi que les produits quaternisés.

16. Utilisation de polymères greffés solubles dans l'eau ou dispersibles dans l'eau selon la revendication 4, **caractérisée en ce que** la base de greffage c4) est choisie parmi des monosaccharides, des oligosaccharides, des polysaccharides, des polysaccharides ayant subi une décomposition oxydative, hydrolytique ou enzymatique, des oligo- ou polysaccharides chimiquement modifiés, et leurs mélanges.

17. Utilisation de polymères selon la revendication 1, **caractérisée en ce que** l'on choisit comme autres comonomères b) dans le groupe formé par: des acides carboxyliques monoéthyléniquement insaturés et leurs sels, des esters, amides et nitriles d'acides carboxyliques monoéthyléniquement insaturés, l'anhydride maléique et ses semi-esters, le chlorure de diallylammonium, des esters vinyliques, le styrène, des alkylstyrènes, des acides sulfoniques insaturés, du N-vinyllactame, des éthers vinyliques, du 1-vinylimidazole et des vinylimidazoles alkylsubstitués ainsi que leurs sels avec des acides carboxyliques ou des acides minéraux ainsi que leurs produits quaternisés, des (méth)acrylates de N,N-dialkylaminoalkyle ainsi que leurs produits quaternisés.

18. Utilisation de polymères selon la revendication 1, **caractérisée en ce que** les polymères sont au moins en partie saponifiés.
